# FASCICULE DE BREVET EUROPEEN

(11) **EP 3 982 824 B1**
(45) Date de publication et mention de la délivrance du brevet: **31.07.2024**
(21) Numéro de dépôt: 20732837.8
(22) Date de dépôt: 11.06.2020
(51) Int. Cl.: A61B 5/01, A61B 5/00, A61B 5/0205, G01K 13/00

(54) **DISPOSITIF DE MESURE DE TEMPERATURES ET SYSTEME DE DETERMINATION D'UNE TEMPERATURE INTERNE PROFONDE D'UN ETRE HUMAIN**
TEMPERATURMESSVORRICHTUNG UND SYSTEM ZUR BESTIMMUNG EINER TIEFEN INNENTEMPERATUR EINES MENSCHEN
TEMPERATURE MEASUREMENT DEVICE AND SYSTEM FOR DETERMINING A DEEP INTERNAL TEMPERATURE OF A HUMAN BEING

(30) Priorité: 11.06.2019 FR 1906200
(43) Date de publication de la demande: 20.04.2022
(73) Titulaire: F2D Medical, 14610 Cairon (FR)
(72) Inventeur: D'ESTAIS, Mathias, 14000 CAEN (FR); FROGER, Benoît, 14150 OUISTREHAM (FR); CORBIN, Jean-Yves, 14480 LE FRESNE CAMILLY (FR); MENARD, Benjamin, 14280 AUTHIE (FR); VAUPRES, Maxime, 14540 GRENTHEVILLE (FR)
(74) Mandataire: IPAZ
(86) Numéro de dépôt international: PCT/EP2020/066186
(87) Numéro de publication internationale: WO 2020/249665

(56) Documents cités:
- WO-A2-2007/021751
- US-A1- 2007 206 655
- US-A1- 2013 218 022
- US-A1- 2018 184 902

## Description

### Domaine technique de l'invention

L'invention concerne le domaine technique des dispositifs de mesure et de détermination de température interne profonde des êtres humains.

Pour un praticien, la valeur de température corporelle significative est la température interne profonde, dite « core température » ou « core body température » ou « CBT » en anglais. Il s'agit de la température des parties internes du corps, incluant ainsi, le sang et les organes vitaux, tels que le coeur, le foie, les reins, les poumons, etc.

La température interne profonde d'un être humain peut être mesurée par de nombreux types de capteurs, comme les sondes invasives destinées à équiper les patients lors des opérations chirurgicales ou dans certains soins intensifs. Il s'agit par exemple des capteurs destinés à être localisés dans l'oesophage passant par la voie orale, ou de capteurs localisés dans la vessie passant par la voie urétrale, ou encore par voie intravasculaire, notamment par des cathéters munis de capteurs thermiques.

Ces différents types de capteurs étant tous invasifs, ils sont difficiles à mettre en oeuvre et peuvent être à l'origine d'une vectorisation de pathogènes dans l'organisme du patient et sont de ce fait réservés à des cas spécifiques. Or la connaissance et le suivi de la température interne profonde est un véritable besoin car la création d'un dispositif non invasif fiable et utilisable notamment en soins hospitaliers courants ou en soins à domicile, ouvrirait de nombreuses possibilités médicales pour établir un diagnostic, pour des protocoles de surveillances ou pour des protocoles d'adaptation des médications aux patients.

Ainsi, des recherches ont été conduites pour obtenir cette donnée sans avoir à mesurer la température interne profonde, mais en inférant la valeur de la température interne profonde à partir d'informations remontées depuis différents capteurs cutanés périphériques.

Pour que cette estimation ou inférence soit précise, un tel dispositif se doit de :
- Prendre en compte que chaque partie du corps est assujettie à des mécanismes de thermorégulation qui donc par extension, lui confèrent une mesure de la température différente des autres parties du corps et de la température interne profonde ;
- Prendre en compte que la température de chaque site est dépendante des conditions locales spécifiques de celui-ci au moment de la mesure. En effet, dès lors que l'on prend la température en périphérie, le site choisi est nécessairement exposé à des conditions bien spécifiques. Par exemple, en procédant à une mesure auriculaire, on comprend bien que cette mesure est impactée si le patient est couché sur l'oreille sur laquelle on prend la mesure ou s'il ne l'est pas, de même, en prenant la mesure sur le bras, on comprend bien que la température mesurée est différente si le bras est couvert par une couette ou s'il ne l'est pas ;
- Prendre en compte que la température au niveau d'un site de mesure dépend d'une activité physique ou biologique spécifique de celui-ci au moment de la mesure, pouvant générer une élévation ou un abaissement de la température du site, potentiellement indépendamment du reste du corps. Par exemple, la température du cerveau et donc de la tête évolue selon l'activité cérébrale, de même la température sous l'aisselle évolue en fonction de l'activité du groupe musculaire du bras. Ces variations de température sont locales mais ont aussi un retentissement sur les autres sites périphériques, néanmoins elles sont compensées par le corps pour qu'elles n'impactent pas directement la température interne profonde. Toute activité physique, mentale, digestive, et certaines réactions biologiques du corps (inflammations locales), est donc susceptible de créer une différence locale trompeuse.

En plus des facteurs précédemment exposés qui influent notablement sur la température interne profonde, la conception concrète du dispositif de mesure fait apparaître d'autres nécessités qui sont toutes aussi importantes à prendre en compte :
- Les contraintes ergonomiques du dispositif porté : s'agissant d'un dispositif de mesure porté sur le corps pendant potentiellement plusieurs jours, on doit veiller à ce que l'inconfort du patient soit le plus limité possible, et que l'utilisateur ressente le moins possible de gêne dans les postures et dans les mouvements qu'il réalise, et inversement, que ces postures et mouvements ne génèrent pas de perturbations sur le bon fonctionnement du dispositif ;
- Les contraintes ergonomiques dans la procédure de pose du dispositif de mesure : on cherche à ce que cette opération soit facile et rapide, et de préférence ménage l'intimité du patient ;
- La difficulté de précision lors de la pose : il est souhaitable que le dispositif de mesure puisse être posé de manière précise et répétable afin de fournir des données robustes ;
- Les variations morphologiques et biologiques inter-individuelles : si l'on considère l'être humain dans son extrême diversité et en prenant en compte les différents stades évolutifs de l'individu, on comprend aisément qu'il sera difficile de créer un dispositif de mesure unique adapté à tous (du nouveau-né à la personne âgée, sain ou pathologique, dont les anorexies et obésités et autres comorbidités). Cependant, il est souhaitable de limiter le nombre de versions, et donc que chaque version ait la plus grande adaptabilité possible

### Etat de la technique

On connaît du document US 2007/206655 A1 un pansement incorporant des capteurs de température.

On connaît du document US 2018/184902 A1 un patch incorporant des capteurs de température. Des dispositifs de mesure de température à proximité d'une aisselle sont également connus des documents US 2013/218022 A1 et WO 2007/021751 A2.

On connaît du document US 2011/243183 un dispositif de mesure de température se présentant sous la forme d'un patch pouvant être apposé sur le corps d'une personne, notamment au niveau du front, de l'arrière de la tête, de la poitrine ou du milieu du dos. Ce patch est destiné à rester en place sur le corps pendant de longues périodes, pour réaliser le suivi continu de la température.

Le dispositif décrit dans ce document comprend un support isolant de la chaleur incluant au moins deux parties de mesure. Chaque partie de mesure comporte elle-même un capteur de température de surface corporelle, situé sur la face interne du support isolant en contact avec le corps du porteur, une couche de contrôle du dégagement de chaleur adjacente à la face externe du support isolant, un capteur de température de l'air extérieur situé sur la face externe de ladite couche, et un capteur intermédiaire de la température à l'interface entre le support isolant et ladite couche.

Les mesures obtenues par ces trois capteurs de température (à savoir : capteur de température de surface corporelle, capteur de température de l'air extérieur et capteur intermédiaire de la température à l'interface) sont utilisées pour la détermination de la température interne profonde du porteur.

Ce dispositif de mesure ne donne pas pleinement satisfaction. Notamment, il fournit des mesures de la température de surface corporelle qui sont fortement dépendantes du positionnement du patch sur le porteur. En outre, l'influence de paramètres extérieurs influant sur les températures mesurées n'est pas prise en compte de façon optimale. Il en résulte des risques d'imprécision de la température interne profonde déterminée.

### Objet de l'invention

Dans le présent exposé de l'invention et dans la description de modes de réalisation de l'invention, on utilisera de manière équivalente les expressions « configuré(e)(s) pour » et « agencé(e)(s) pour ».

L'invention a pour but de remédier à tout ou partie des inconvénients précités en permettant de déterminer une température interne profonde d'un être humain de façon précise, fiable et efficace, en vue, in fine, d'améliorer le suivi et le traitement des patients.

A cet effet, et selon un premier aspect, l'invention concerne un dispositif de mesure d'une pluralité de températures en vue de la détermination d'une température interne profonde d'un être humain selon la revendication 1.

Selon une définition générale de l'invention, le dispositif de mesure comprend :
- au moins trois capteurs de température cutanée configurés pour mesurer une température cutanée du porteur, les capteurs de température cutanée étant positionnés sur ou au voisinage de la première face du dispositif de mesure, dans une première zone du dispositif de mesure, et s'étendant sensiblement sur au moins une partie d'une ligne périphérique du dispositif de mesure en configuration portée ; de préférence :
   * les au moins trois capteurs de température cutanée sont agencés pour être en contact direct de la peau du bras du porteur sans couche intermédiaire entre les au moins trois capteurs de température cutanée et la peau du porteur; et/ou
   * la première face du dispositif de mesure est agencée pour être en contact direct de la peau du bras du porteur sans couche intermédiaire entre la première face et la peau du bras du porteur, et les au moins trois capteurs de température cutanée sont situés sur cette première face en étant orientés vers l'extérieur du dispositif selon l'invention ;
- au moins un capteur de température cavitaire configuré pour mesurer une température dans ou au voisinage de ladite aisselle du porteur, le capteur de température cavitaire étant agencé sur ou au voisinage de la deuxième face du dispositif de mesure, dans une deuxième zone du dispositif de mesure ; de préférence :
   * l'au moins un capteur de température cavitaire est agencé pour être en contact direct de l'aisselle du porteur sans couche intermédiaire entre l'au moins un capteur de température cavitaire et l'aisselle du porteur; et/ou
   * la deuxième face du dispositif de mesure est agencée pour être en contact direct de l'aisselle du porteur sans couche intermédiaire entre la deuxième face et l'aisselle du porteur, et l'au moins un capteur de température cavitaire est situé sur cette deuxième face en étant orienté vers l'extérieur du dispositif selon l'invention ;
   ladite première zone des capteurs de température cutanée et ladite deuxième zone du ou des capteurs de température cavitaire étant agencées au moins en partie en regard l'une de l'autre ou étant sensiblement adjacentes, en projection dans un plan orthogonalement auxdites première et deuxième faces.

Ainsi, le dispositif de mesure peut être enroulé autour d'un bras du porteur, de préférence en épousant la forme de ce bras. De plus, le positionnement des capteurs de température cutanée sur ou au voisinage de la première face du dispositif leur permet de s'appliquer au plus près de la peau du porteur. L'invention permet ainsi la mesure des températures de façon particulièrement précise et pertinente à partir des différents capteurs. La localisation de ces différents capteurs est apte à fournir les données nécessaires à un algorithme pour inférer la température interne profonde. En outre, le dispositif de mesure peut rester en place facilement sur le bras du porteur, et il peut y demeurer le temps nécessaire à une prise de mesure de température utile au diagnostic et au suivi au long cours du patient.

Le nombre de capteur(s) de température cavitaire est de préférence inférieur au nombre de capteurs de température cutanée.

Lorsqu'il est en place, le dispositif de mesure est placé sensiblement transversalement sur le bras du porteur, selon une ligne périphérique du bras. Il est précisé que le dispositif de mesure peut être enroulé sur moins d'un tour, ou au contraire sur plus d'un tour complet, par exemple en se recouvrant lui-même ou en formant une hélice autour du bras du porteur. Par exemple, dans la configuration portée, le dispositif de mesure peut s'étendre angulairement (i.e. autour de l'axe Z du cylindre) sur au moins 90°, de préférence au moins 180°, encore mieux au moins 270°, ou plus de 360°. On note qu'un dispositif de mesure donné, lorsqu'il est porté, peut couvrir un enroulement angulaire plus ou moins important suivant le diamètre du bras du porteur.

De façon concrète, la ligne périphérique correspond à la direction d'enroulement du dispositif de mesure ; elle peut correspondre à la longueur du dispositif de mesure. La ligne périphérique peut typiquement être située dans un plan orthogonal à l'axe (Z) du cylindre formé par le dispositif de mesure, c'est-à-dire dans un plan orthogonal à l'axe du bras du porteur.

Le positionnement du dispositif de mesure sur le bras présente un certain nombre d'avantages : il est positionné affleurant sur une couche de chair et de graisse faible, y compris dans une moindre mesure chez les personnes obèses, ce qui permet une mesure non faussée (autrement dit, une mesure plus proche de la valeur de la température des artères). En outre, ce positionnement présente des perspectives de confort acceptables pour le porteur, et est raisonnablement accessible par un tiers pour sa pose ou son retrait, ou pour consulter l'état de la zone du corps sur laquelle est installé le dispositif de mesure, tout en limitant le caractère invasif de ces opérations pour le porteur. Un autre avantage est que l'ensemble des capteurs est regroupé sur un seul dispositif si bien que son installation est simple et rapide.

Selon une caractéristique de l'invention, le dispositif de mesure se présente sous la forme d'un brassard à porter sur le bras d'un être humain, préférentiellement à proximité du creux axillaire aux fins d'obtenir une valeur de la température de la peau au niveau de l'artère brachiale. Cet emplacement à proximité de l'extrémité de la tête humérale permet une forte proximité entre un capteur de température posé de manière adéquate en surface de la peau sur la face interne du bras, et une section importante de l'artère brachiale. Avantageusement, le brassard peut être positionné tout autour du bras ou ne couvrir qu'une portion de la section transversale du bras c'est-à-dire n'être que partiellement entouré autour du bras du porteur.

Dans la présente invention, on entend par les termes « température cutanée » la température mesurée au plus proche de la peau du porteur et préférentiellement la température mesurée sur la peau.

Les capteurs de température cutanée sont de préférence positionnés dans une zone irriguée par un flux sanguin important, dont la section ciblée est la plus proche du coeur, et avantageusement également proche des organes vitaux, pour éviter de mesurer une température très dépendante de la température extérieure.

En prévoyant que les capteurs de température cutanée s'étendent sur au moins une partie d'une ligne périphérique du dispositif de mesure en configuration portée, on peut obtenir une pluralité de températures cutanées mesurées dans la zone souhaitée, en l'occurrence autour de l'artère brachiale, et le plus possible en regard de celle-ci. Ceci permet d'obtenir des mesures significatives de la température de l'artère brachiale et ce malgré des phénomènes de variabilité perturbant la mesure et que nous décrirons dans les paragraphes suivants. L'obtention de ces mesures significatives de la température de l'artère brachiale est une donnée importante dans l'algorithme d'inférence de la température interne profonde.

L'avantage d'avoir un dispositif de mesure comprenant une pluralité de capteurs de température cutanée, plus précisément au moins trois capteurs de température cutanée, résulte du constat que le point le plus chaud de la peau est difficile à localiser et susceptible de se déplacer en fonction du temps. Plus précisément, le point le plus chaud est d'abord difficile à localiser car les variabilités interpersonnelles sont importantes et le différentiel de température dont il s'agit est très faible (à cet endroit du corps, le différentiel entre deux points de la circonférence écartés de 1 cm est souvent de moins de 0,2°). Ce point le plus chaud est susceptible de se déplacer au fil du temps par différents phénomènes : de par une activité physique en particulier une activité du biceps ; de par une posture créant une situation géométrique particulière par rapport à une source chaude ou froide, et en particulier par rapport au tronc; enfin, de par la situation du bras du porteur qui peut être vertical, incliné ou horizontal, et plus ou moins conformé par un objet, ce qui impacte sur la conduction et sur la position interne des chairs et de l'artère et qui influe directement aussi sur la diffusion de la chaleur en provenance de l'artère au travers des chairs jusqu'à la zone des capteurs.

Il est donc plus judicieux d'avoir plusieurs points de mesure cutanée pour disposer de la séquence des températures de cette zone et pour analyser leurs évolutions dans le temps. Cette pluralité de capteurs de température cutanée forme un groupe de capteurs cutanés. En outre, l'utilisation d'une pluralité de capteurs permet de recouper la donnée de température cutanée et de l'affiner pour plus de précision et de fiabilité des données fournies à l'algorithme pour la détermination de la température interne profonde du porteur. On note que cet avantage s'obtient sans augmentation majeure du prix puisque les capteurs de température sont de coût raisonnable.

On peut prévoir de préférence au moins cinq capteurs de température cutanée, par exemple sept capteurs de température cutanée. Augmenter le nombre de capteurs de température cutanée permet d'augmenter la précision de la température déterminée à partir des mesures de températures cutanées en différents points.

Préférentiellement, le dispositif recueille en permanence les températures issues des capteurs cutanés et, dans une première étape, on s'intéresse à la valeur de la température mesurée la plus élevée parmi les capteurs de température cutanée. En effet, on constate qu'il est possible que la valeur de la température mesurée la plus élevée soit mesurée par un premier capteur à un temps t et par un deuxième capteur à un temps t+n, notamment lorsque la posture change ou lorsque l'activité du porteur varie dans le temps, entraînant une hausse spécifique de la température musculaire.

Dans une deuxième étape, on s'intéresse à contrôler le fait que la température la plus haute n'est pas localisée parmi les capteurs de température cutanée situés à des positions extrêmes parmi la pluralité de ces capteurs. Cela donne une indication sur la bonne adaptation du dispositif de mesure au porteur. Ainsi, si la température la plus haute est mesurée par un capteur de température cutanée situé à une des deux positions extrêmes, on peut légitimement craindre que la température la plus haute mesurable sur la périphérie ne soit mesurée par aucun des capteurs du groupe de capteurs. Si de telles conditions se prolongent, on risque bien de ne pas obtenir de données vraiment exploitables concernant la température réelle de l'artère et donc de ne pas pouvoir inférer avec précision la température interne profonde. On note qu'une mauvaise adaptation du dispositif de mesure au porteur peut en particulier être observée suite à un mauvais positionnement du dispositif, ou suite à un glissement du dispositif au long de la période de mesures, ou suite à des conditions extrêmes d'exercice ou d'environnement du dispositif, ou encore suite à une morphologie particulière du porteur. Quelle qu'en soit la cause, si ces conditions se prolongent, elles sont susceptibles de mettre en péril la qualité des mesures et peuvent déclencher une alerte sur le dispositif.

Pour éviter cela on a aussi constaté empiriquement qu'il est préférable que les capteurs couvrent une longueur suffisante, par exemple de l'ordre d'au moins 50 mm pour un bras adulte. Ceci permet de limiter le risque de se retrouver dans la situation de la température la plus haute localisée sur un des capteurs extrêmes.

Pour ce faire, on a constaté empiriquement qu'une distance d'environ 10 mm entre deux capteurs adjacents permet de couvrir correctement la zone et d'éviter des erreurs significatives.

La mesure de la température cutanée doit être très précise et très fiable. On a ainsi privilégié des capteurs de température spécialisés pour la santé humaine, intégrant un convertisseur analogique-numérique haute résolution.

Dans la présente invention, on entend par les termes « température cavitaire », la température dans une cavité ouverte du porteur en l'espèce les environs d'une aisselle. La température cavitaire est globalement et majoritairement générée par le corps du porteur lui-même, mais peut aussi résulter d'autres sources de température comme le corps d'une autre personne serrée contre soi, une poche rafraîchissante ou chauffante, le soleil, etc. Le dispositif de mesure selon l'invention peut comporter une pluralité de capteurs de température cavitaire.

En fonctionnement du dispositif de mesure, il peut y avoir des sources de chaleur ou des sources froides qui provoquent une élévation ou une diminution trompeuse de la température du dispositif de mesure et de la température mesurée.

Des sources similaires peuvent aussi engendrer une élévation ou une diminution de la température de la zone du corps aux alentours des capteurs de température cutanée, mais cette élévation ou diminution bien réelle de la température de cette partie du corps est souvent aussi trompeuse car elle n'est souvent que superficielle et n'a en réalité aucune ou peu de conséquence sur la température interne profonde du porteur.

Parmi ces sources, on note en tout premier lieu le tronc du corps du porteur lui-même qui peut chauffer ou refroidir le dispositif de mesure par conduction, radiation, et éventuellement convection ; toute autre source chaude ou froide extracorporelle telle que le corps d'un autre individu, une pièce d'habillement ou un sac, un support sur lequel le porteur est installé (tel qu'un matelas, une rambarde), l'air, une poche rafraîchissante ou chauffante, le soleil, etc.

L'usage d'au moins un capteur de température cavitaire au sein du dispositif de mesure permet de tenir compte spécifiquement des variations de températures induites par le tronc et plus généralement de tenir compte de la présence d'une source chaude ou d'une source froide extérieure et d'utiliser la valeur mesurée dans les calculs pour prendre en compte ces situations et bien faire la différence entre un changement de température extérieure (exogène) et un changement de température du corps lui-même (endogène) afin d'obtenir une température interne profonde plus pertinente.

En outre, cette mesure de la ou des températures cavitaires permet de tenir compte de la situation du porteur. Ainsi, si la température cavitaire est nettement inférieure à la température cutanée, il est considéré que cette cavité - ici l'aisselle - est assez ouverte, et donc que le membre du porteur - ici le bras - est assez écarté de son corps ou d'un corps générant de la chaleur. A l'inverse, si la température cavitaire est sensiblement égale à la température cutanée, on peut en déduire que le capteur de température cavitaire est a priori à proximité de la peau du porteur. Ces éléments ramenés ici à des données simples pour l'explication sont en substance des variables continues qui sont des informations d'entrée importantes pour l'algorithme du système, et permettent de déterminer la température interne profonde avec une précision et une fiabilité accrues.

Ces éléments sous-tendent une grande complexité. Ainsi par exemple, si l'on se figure la présence d'un pyjama chaud avec une cavité presque refermée et une certaine transpiration sous l'aisselle, cette configuration comporte de multiples effets thermiques : isolation relative du tronc vers le dispositif par la matière du pyjama, radiation de l'aisselle vers le dispositif, évapo-transpiration naturelle de l'aisselle et évaporation au travers du pyjama, convection naturelle entre le pyjama et la peau, etc. Il faut comprendre que le ou les capteurs cavitaires ont pour objet de donner une information globale à l'ensemble de ces phénomènes afin de corriger les mesures réalisées par les capteurs peau. Ils n'ont pas pour vocation d'être très précis. Ils opèrent un correctif.

Le dispositif de mesure peut comporter plusieurs capteurs de température cavitaire. Ces capteurs peuvent être au nombre d'au moins trois.

L'exigence de précision sur les mesures de température cavitaire est moins forte que pour les mesures de températures cutanées. On peut donc choisir des capteurs plus simples, tels que des thermocouples ou des thermistances, qui sont plus simples à mettre en oeuvre et moins coûteuses.

Par « zone » dans laquelle s'étendent les capteurs, on entend une surface, de préférence sensiblement rectangulaire, qui englobe l'ensemble des capteurs considérés, au plus près de ceux-ci.

Comme indiqué précédemment, la première zone, dans laquelle sont agencés les capteurs de température cutanée, et la deuxième zone, dans laquelle sont agencés les capteurs de température cavitaire, peuvent être au moins en partie en regard l'une de l'autre en projection dans un plan orthogonalement auxdites première et deuxième faces. Cela signifie que, dans la direction de l'épaisseur du dispositif de mesure, ces première et deuxième zones sont totalement superposées, ou qu'il existe un recouvrement partiel de ces première et deuxième zones.

En variante, les première et deuxième zones peuvent être sensiblement adjacentes en projection dans un plan orthogonalement auxdites première et deuxième faces. Cela signifie que, vu dans ledit plan de projection, les première et deuxième zones ne sont pas superposées mais sont décalées l'une par rapport à l'autre en restant proches l'une de l'autre, voire en étant jointives. Par « proches », on entend que la distance de séparation entre les première et deuxième zones selon la projection précédemment considérée (i.e. la longueur du plus petit segment reliant ces projections de la première zone et de la deuxième zone) est inférieure à 3 cm, de préférence inférieure à 1,5 cm, voire inférieure à 0,8 cm, voire même inférieure à 0,5 mm. Vues dans ledit plan de projection, les première et deuxième zones peuvent être décalées parallèlement à l'axe (Z), ou orthogonalement à l'axe (Z), ou les deux.

Selon une réalisation possible, le dispositif de mesure est souple et configuré pour pouvoir être déformé, de préférence par enroulement, entre une configuration non-portée et la configuration portée. Cette souplesse permet au dispositif de mesure d'une part d'être facilement mis en place, et d'autre part de bien épouser la forme du bras sur lequel il est placé. La qualité des mesures de température cutanée effectuées s'en trouve considérablement améliorée.

De façon concrète, de par sa souplesse, le dispositif de mesure peut s'adapter à différentes formes convexes ou concaves du corps du porteur. Il est notamment positionnable sur des bras avec des biceps saillants, des bras dénutris, des bras de personnes en surcharge pondérale, des bras de personnes âgées avec une peau fine et en perte d'élasticité, etc. En outre, le dispositif de mesure est positionnable sur un porteur occupant différentes positions, par exemple en position allongée, ce qui peut générer des plis.

De préférence, le dispositif de mesure est sensiblement non extensible, en particulier dans sa direction longitudinale (correspondant à la périphérie du bras), afin d'éviter tout effet de garrot sur le porteur.

Selon la présente invention, le dispositif de mesure est configuré pour pouvoir être dans une configuration non portée dans laquelle il est sensiblement plan. En variante , variante non couverte par la présente invention, le dispositif de mesure pourrait, dans sa configuration non portée, être déjà enroulé en un cylindre. Le diamètre de ce cylindre peut être plus grand qu'en configuration portée, un enroulage supplémentaire étant donc nécessaire pour placer le dispositif de mesure en configuration portée. De façon alternative, le diamètre de ce cylindre pourrait être plus faible qu'en configuration portée, pour autant qu'il n'y ait pas ou peu d'effet de serrage ou d'effet de garrot généré. Avantageusement, le diamètre du dispositif de mesure en configuration portée est réglable et ajustable au bras du porteur.

Le dispositif de mesure selon l'invention comprend en outre au moins un capteur de température proximale configuré pour mesurer une température environnante à proximité immédiate du bras du porteur, le ou les capteurs de température proximale étant agencé(s) sur ou au voisinage de la deuxième face du dispositif de mesure. Le capteur de température proximale - ou le centre du groupe de capteurs de température proximale - peut être décalé angulairement (i.e. autour de l'axe Z du cylindre) par rapport au capteur de température cavitaire ou à l'axe du groupe de capteurs de température cavitaire, en configuration portée du dispositif de mesure, d'au moins 90°. De préférence, le décalage angulaire précité est d'environ 180°. En d'autres termes, le capteur de température proximale est de préférence positionné de façon sensiblement diamétralement opposée à l'au moins un capteur de température cavitaire, en configuration portée du dispositif de mesure. Une telle disposition permet d'obtenir une mesure fiable de la température proximale, en minimisant l'influence de la chaleur du corps du porteur.

En configuration non-portée, la distance entre un capteur de température cutanée et l'au moins un capteur de température proximale est supérieure à 10 cm et de préférence inférieure à 20 cm.

De préférence, l'au moins un capteur de température proximale est situé sur la deuxième face en étant orienté vers l'extérieur du dispositif selon l'invention.

Autrement dit, on veillera de préférence à implanter les capteurs de température proximale pour qu'ils soient disposés sur la face du bras qui est tournée vers l'extérieur, donc non tournée vers le tronc, alors que le ou les capteurs de température cavitaire sont pour leur part disposés sur la face du bras qui est tournée vers le tronc.

Dans la présente invention, on entend par les termes « température proximale » la température mesurée dans l'environnement immédiat autour du dispositif de mesure, procurant une information de la température dans l'environnement immédiat du porteur. Cette température varie en particulier en fonction des pertes de calories par le porteur, des apports ou pertes de calories de l'environnement et des caractéristiques des vêtements, draps, couvertures, utilisés par le patient.

La disposition du capteur de température proximale, ou le centre du groupe de capteurs de température proximale, orienté vers l'extérieur du corps du porteur, et sensiblement à l'opposé du groupe de capteurs de température cavitaire, ou d'au moins un des capteurs de température cutané, permet d'obtenir une valeur de la température proximale subissant de manière limitée l'influence des apports thermiques du corps du porteur. Bien entendu, ce positionnement dépend de la circonférence du bras du porteur, le capteur de température proximale pouvant ainsi être plus vers l'avant (c'est-à-dire le ventre) ou plus vers l'arrière (c'est-à-dire le dos) du corps du porteur.

Par ailleurs, dans le cas d'un positionnement du dispositif de mesure sur le bras, à proximité de l'aisselle, la zone à l'opposé de l'artère brachiale est moins vascularisée et davantage graisseuse. De ce fait, elle est moins réactive aux variations de température internes du corps. Ainsi la température mesurée par le capteur de température proximale délivre une certaine mesure de l'environnement thermique à proximité immédiate du corps dans sa globalité.

En d'autres termes, en configuration non-portée, la distance entre un capteur de température cutanée - par exemple le centre des capteurs de température cutanée - et le au moins un capteur de température proximale - par exemple le centre des capteurs de température proximale - est sensiblement égale à la moitié de la circonférence du bras du porteur, de préférence supérieure à 10 cm et/ou inférieure à 20cm, par exemple entre 10 et 20 cm.

Une valeur angulaire peut être traduite en une mesure périphérique pour un bras donné et réciproquement. Par exemple, si l'on considère que 28 cm est le périmètre moyen du bras d'un adulte à la naissance de l'aisselle, on peut avantageusement choisir que le centre des capteurs de température cutanée soit éloigné de 14 cm du centre des capteurs de température proximale. Dans ces conditions, les centres de ces deux groupes de capteurs sont ainsi placés de manière exactement opposée pour un porteur ayant un bras de 28 cm de périmètre, ce qui est une situation favorable. Si l'on équipe de ce même dispositif de mesure un porteur ayant un bras de taille différente, par exemple un bras de périmètre 20 cm, le point exactement opposé à son artère brachiale est situé à 10 cm. En supposant que le dispositif de mesure est installé avec le centre du groupe de capteurs de température cutanée au-dessus de l'artère, la position du centre du groupe de capteurs de température proximale selon la configuration précédente est alors à 4 cm du point cible théorique, créant un décalage acceptable vers l'avant ou vers l'arrière, selon le sens de pose du dispositif de mesure. On peut aussi exprimer ces valeurs angulairement : le centre des capteurs de température cutanée forme avec le centre des capteurs de température proximale un angle de 14 cm / 20 cm x 360° = 252°, et le décalage vers l'avant ou vers l'arrière est de 4 cm / 20 cm x 360°, soit 72°.

Le dispositif de mesure peut comporter une pluralité de capteurs de température proximale, afin d'obtenir une estimation au plus juste de la température autour du corps. Selon une caractéristique de l'invention, la pluralité de capteurs de température proximale comprend au moins deux capteurs de température, préférentiellement alignés selon un axe longitudinal du dispositif de mesure. On peut prévoir d'équiper le dispositif de mesure d'au moins au moins trois capteurs de température proximale. Une fonction du ou des capteurs de température proximale est de fournir une mesure de température permettant de corriger les mesures de température effectuées au niveau de l'artère brachiale, par les capteurs de température cutanée, cette fonction pouvant être assurée de façon satisfaisante par un unique capteur de température proximale.

L'exigence de précision sur les mesures de proximale est moins forte que pour les mesures de températures cutanées. On peut donc choisir des capteurs plus simples, tels que des thermocouples ou des thermistances, qui sont plus simples à mettre en oeuvre et moins coûteuses.

Le dispositif de mesure peut en outre comprendre au moins un capteur de données physico-chimiques complémentaires. Selon le type de capteur concerné, il peut être agencé sur ou au voisinage de la première face du dispositif de mesure (dans ce cas, l'au moins un capteur de données physico-chimiques est de préférence situé sur la première face en étant orienté vers l'extérieur du dispositif selon l'invention et/ou est agencé pour être en contact direct de la peau du bras du porteur sans couche intermédiaire entre l'au moins un capteur de données physico-chimiques et la peau du porteur), ou bien sur ou au voisinage de la deuxième face du dispositif de mesure (dans ce cas, l'au moins un capteur de données physico-chimiques est de préférence situé sur la deuxième face en étant orienté vers l'extérieur du dispositif selon l'invention).

L'adjonction de fonctions via des capteurs de données physico-chimiques enrichit les prises de mesures réalisées par le dispositif de mesure selon l'invention en les contextualisant, ce qui permet d'écarter ou d'inclure du processus d'inférence des mesures de températures qui seraient dues à des causes environnementales ou d'états particuliers dans lesquels se trouve le porteur.

Selon l'invention, le ou les capteurs de données physico-chimiques complémentaires peut être un photoplethysmographe configuré pour mesurer la fréquence cardiaque et l'oxymétrie du porteur. Avantageusement, le photoplethysmographe peut être positionné sur la première face du dispositif de mesure et est destiné à venir au contact de la peau du porteur. Le photoplethysmographe peut être agencé à proximité immédiate du capteur central de température cutanée. Ce positionnement est avantageux car il permet d'augmenter la qualité de mesure, du fait d'une plus grande irrigation de la peau sur cette zone.

Pour respecter la disposition des capteurs de température cutanée organisés selon au moins une partie d'une ligne périphérique du dispositif de mesure en configuration portée, on peut disposer le photopléthysmographe au niveau du centre du groupe de capteurs de température cutanée, mais déporté suffisamment pour que les deux types de capteurs en présence ne se perturbent pas mutuellement. De ce fait, en installant le dispositif sur le porteur avec le capteur cutané central au-dessus de l'artère brachiale, le photopléthysmographe sera ainsi situé de manière adaptée, au-dessus de l'artère, et pourra mesurer efficacement la fréquence cardiaque ou l'oxymétrie.

Alternativement ou en complément, le capteur de données physico-chimiques complémentaires peut être un galvanomètre configuré pour mesurer le niveau de transpiration et de perspiration sécrété par le corps du porteur. Le galvanomètre peut comprendre au moins deux capteurs mesurant de faibles intensités de courant électrique et permettant de qualifier les excrétions du corps et de déterminer la composition physico-chimique desdites excrétions.

Les capteurs du galvanomètre sont positionnés sur la première face du dispositif de mesure, en contact avec la peau du porteur. Préférentiellement, les capteurs du galvanomètre sont positionnés aux environs du centre du groupe de capteurs de température proximale, mais situés sur la face opposée du dispositif. La sudation dans cette zone est le reflet d'une sudation générale du corps, ce qui est intéressant pour avoir une information générale sur l'état du porteur.

Alternativement ou en complément, un capteur galvanométrique peut être localisé aux environs du groupe de capteurs de température cutanée, ce qui permet de capter la sudation spécifie de la zone aisselle.

Alternativement ou en complément, le capteur de données physico-chimiques complémentaires peut être un accéléromètre trois axes, un gyroscope ou un GPS, configuré pour obtenir une information sur l'activité cinématique du corps du porteur.

Selon une réalisation possible, ladite première zone dans laquelle sont agencés les capteurs de température cutanée est allongée et s'étend le long d'une direction longitudinale du dispositif de mesure, les capteurs de température cutanée étant disposés de façon alignée ou en quinconce dans ladite zone allongée. Ceci peut également s'appliquer aux capteurs de température cavitaire.

Une disposition en quinconce permet de densifier le nombre de capteurs sur une portion réduite, et ainsi d'obtenir une mesure plus précise et plus fiable tout en limitant les transmissions de calories d'un capteur à l'autre. En outre, grâce à cette disposition en quinconce, chaque capteur va recueillir une mesure peu significativement différente par rapport à un positionnement de façon alignée.

Le au moins un capteur de température cavitaire peut être agencé en regard de ladite première zone des capteurs de température cutanée, de préférence de façon sensiblement centrée, selon la direction longitudinale.

Selon un mode de réalisation, le ou les capteurs de température cavitaire peuvent être en regard de ladite zone sans nécessairement être en regard d'un capteur de température cutanée.

Selon un autre mode de réalisation, au moins un capteur de température cavitaire est agencé en regard d'au moins un capteur de température cutanée et préférentiellement en regard d'un capteur central de température cutanée, lorsque le nombre de capteurs de température cutanée est impair. On choisira préférentiellement que ce capteur soit un peu désaxé par rapport à la ligne des capteurs de température cutanée afin que les capteurs de température cutanée soient tous implantés dans un environnement mécanique très similaire (contexte de matériaux similaires pour avoir un comportement d'isolation et de transmission thermique similaire avec l'extérieur et entre eux) et afin de limiter les perturbations de mesure du fait du capteur de température cavitaire. Une telle disposition permet de relever avec précision les données de la température cutanée dans la zone de l'artère brachiale.

Selon un mode de réalisation, on peut prévoir deux capteurs de température proximale agencés à chaque extrémité du dispositif de mesure, selon la direction longitudinale, et préférentiellement agencés symétriquement par rapport au capteur central du groupe des capteurs de température cutanée. Selon cet agencement, le groupe de capteurs de température proximale est réparti sur deux sites qui peuvent être très espacés l'un de l'autre, en particulier lorsque le dispositif de mesure est sensiblement plan en configuration non portée. Toutefois lorsque le dispositif de mesure est en configuration portée, les capteurs de température proximale se retrouvent à proximité l'un de l'autre suite à l'enroulement du dispositif de mesure.

Selon une réalisation possible, chaque capteur de température cavitaire est positionné en regard d'un capteur de température cutanée. L'axe des capteurs de température cutanée peut être décalé on non de l'axe des capteurs de température cavitaire.

Préférentiellement, les capteurs de température cavitaire sont répartis sur l'ensemble de la zone du dispositif de mesure dans laquelle sont agencés les capteurs de température cutanée.

Selon une caractéristique de l'invention, les capteurs de température cutanée et/ou les capteurs de température cavitaire et/ou les capteurs de température proximale sont répartis sur au moins une portion du dispositif de mesure, ayant une dimension dans la direction longitudinale d'au moins 45 mm.

La configuration des capteurs, par exemple 7 répartis sur 50 mm, peut évoluer selon le diamètre du dispositif de mesure. Par exemple, on peut prévoir 5 capteurs répartis sur 35 mm pour un bras de bébé ou 11 capteurs répartis sur 80 mm pour un porteur adulte ayant un très fort IMC.

Selon une caractéristique de l'invention, les capteurs de température cutanée et/ou les capteurs de température cavitaire et/ou les capteurs de température proximale sont alignés sur un axe longitudinal du dispositif de mesure.

Selon une caractéristique de l'invention, les capteurs de température cutanée et/ou les capteurs de température cavitaire et/ou les capteurs de température proximale sont espacés régulièrement avec un entraxe identique.

Selon une caractéristique de l'invention, les capteurs de température cutanée et/ou les capteurs de température cavitaire et/ou les capteurs de température proximale sont agencés en quinconce ou en biais sur les faces respectives du dispositif de mesure.

Selon une réalisation possible, le dispositif de mesure se présente sous la forme d'une bande allongée dans une direction longitudinale (X) qui, dans la configuration portée, correspond sensiblement à une ligne périphérique du dispositif de mesure, la bande présentant :
- une longueur, selon la direction (X), de préférence supérieure à 12 cm, encore mieux supérieure à 15 cm, voire supérieure à 20 cm ;
- une largeur, selon une direction qui est sensiblement parallèle à l'axe (Z) du cylindre en configuration portée, de préférence inférieure à 6 cm, encore mieux inférieure à 4 cm, par exemple de l'ordre de 3 cm ;
et la bande possédant un axe longitudinal médian, un axe transversal médian, un bord supérieur et un bord inférieur (lorsque le dispositif de mesure est porté, le porteur étant dans la position anatomique de référence, c'est-à-dire en particulier debout, avec les bras le long du corps).

Au moins une face parmi la première face et la deuxième face peut comporter une surface plane en configuration non-portée.

Les capteurs de température cutanée et/ou le au moins un capteur de température cavitaire peuvent être disposés sensiblement le long de l'axe longitudinal médian de la bande.

En variante, les capteurs de température cutanée et/ou le au moins un capteur de température cavitaire peuvent être décalés en direction du bord supérieur par rapport à l'axe longitudinal médian de la bande. Un avantage de cette disposition est que les capteurs de température cavitaire peuvent être placés au plus près de l'aisselle. Ceci peut également être intéressant pour les capteurs de température cutanée. En effet, l'artère brachiale est affleurante sous la peau du bras à proximité de l'aisselle, tandis qu'elle localisée plus en profondeur dans le bras à distance de l'aisselle.

Le dispositif de mesure peut en outre comprendre :
- une nappe allongée portant les capteurs de température cutanée et le au moins un capteur de température cavitaire ;
- un boîtier contenant une carte électronique et une batterie connectées à la nappe allongée, le boîtier portant de préférence le au moins un capteur de température proximale et/ou portant de préférence le au moins un capteur de données physico-chimiques complémentaires, lorsque ces capteurs sont présents ;
le boîtier et la nappe allongée étant englobés, par exemple par surmoulage, dans une matière souple.

Ladite matière souple peut être un élastomère ou un silicone de qualité médicale, ce qui permet d'améliorer le confort du porteur du dispositif de mesure et d'assurer un contact homogène sans décollement avec la peau du porteur. Dans la présente invention, on entend par « silicones de qualité médicale » des matériaux testés pour une biocompatibilité et appropriés pour être utilisés pour des applications médicales.

Par exemple, on peut mettre en oeuvre une injection en silicone médical grade 35 ShoreA. Selon cet exemple, le dispositif de mesure peut être d'une épaisseur sensiblement égale à 2,5 mm, l'électronique comprenant les capteurs étant intégrée en nappe Kapton de 10 mm de large et de 0.20 mm d'épaisseur.

La nappe forme un support pour les capteurs et intègre aussi des organes de connexion électronique.

Le dispositif de mesure peut également comporter un revêtement de propreté, avantageusement lavable, sur sa première face et/ou sa deuxième face. Il peut en outre comprendre une portion adhésive destinée à être collée sur le bras du porteur.

Selon une réalisation possible, le dispositif de mesure comprend :
- un premier jeu de capteurs comportant :
   * au moins trois capteurs de température cutanée qui sont positionnés sur ou au voisinage de la première face du dispositif de mesure, dans une première zone du premier jeu de capteurs, et qui s'étendent sur une première longueur; de préférence, les au moins trois capteurs de température cutanée du premier jeu sont agencés pour être en contact direct de la peau du bras du porteur sans couche intermédiaire entre les au moins trois capteurs de température cutanée du premier jeu et la peau du porteur, et/ou la première face du dispositif de mesure est agencée pour être en contact direct de la peau du bras du porteur sans couche intermédiaire entre la première face et la peau du bras du porteur, et les au moins trois capteurs de température cutanée du premier jeu sont situés sur cette première face en étant orientés vers l'extérieur du dispositif selon l'invention ;
   * au moins un capteur de température cavitaire qui est agencé sur ou au voisinage de la deuxième face du dispositif de mesure, dans une deuxième zone du premier jeu de capteurs du dispositif de mesure qui est au moins en partie en regard de la première zone du premier jeu de capteurs, ou sensiblement adjacente à celle-ci, en projection dans un plan orthogonalement auxdites première et deuxième faces ; de préférence, l'au moins un capteur de température cavitaire du premier jeu est agencé pour être en contact direct de l'aisselle du porteur sans couche intermédiaire entre l'au moins un capteur de température cavitaire du premier jeu et l'aisselle du porteur, et/ou la deuxième face du dispositif de mesure est agencée pour être en contact direct de l'aisselle du porteur sans couche intermédiaire entre la deuxième face et l'aisselle du porteur, et l'au moins un capteur de température cavitaire du premier jeu est situé sur cette deuxième face en étant orienté vers l'extérieur du dispositif selon l'invention ;
- un deuxième jeu de capteurs comportant :
   * au moins trois capteurs de température cutanée qui sont positionnés sur ou au voisinage de la deuxième face du dispositif de mesure, dans une première zone du deuxième jeu de capteurs, et qui s'étendent sur une deuxième longueur de préférence différente de la première longueur; de préférence, les au moins trois capteurs de température cutanée du deuxième jeu sont agencés pour être en contact direct de la peau du bras du porteur sans couche intermédiaire entre les au moins trois capteurs de température cutanée du deuxième jeu et la peau du porteur, et/ou la deuxième face du dispositif de mesure est agencée pour être en contact direct de la peau du bras du porteur sans couche intermédiaire entre la deuxième face et la peau du bras du porteur, et les au moins trois capteurs de température cutanée du deuxième jeu sont situés sur cette deuxième face en étant orientés vers l'extérieur du dispositif selon l'invention ;
   * au moins un capteur de température cavitaire qui est agencé sur ou au voisinage de la première face du dispositif de mesure, dans une deuxième zone
   du deuxième jeu de capteurs du dispositif de mesure qui est au moins en partie en regard de la première zone du deuxième jeu de capteurs, ou sensiblement adjacente à celle-ci, en projection dans un plan orthogonalement auxdites première et deuxième faces. De préférence, l'au moins un capteur de température cavitaire du deuxième jeu est agencé pour être en contact direct de l'aisselle du porteur sans couche intermédiaire entre l'au moins un capteur de température cavitaire du deuxième jeu et l'aisselle du porteur, et/ou la première face du dispositif de mesure est agencée pour être en contact direct de l'aisselle du porteur sans couche intermédiaire entre la première face et l'aisselle du porteur, et l'au moins un capteur de température cavitaire du deuxième jeu est situé sur cette première face en étant orienté vers l'extérieur du dispositif selon l'invention ;

Le dispositif est de préférence agencé pour utiliser ou activer un seul jeu de capteurs à la fois parmi le premier jeu de capteurs et le deuxième jeu de capteurs.

De préférence :
- le nombre de capteur(s) de température cavitaire du premier jeu est inférieur au nombre de capteurs de température cutanée du premier jeu, et
- le nombre de capteur(s) de température cavitaire du deuxième jeu est inférieur au nombre de capteurs de température cutanée du deuxième jeu.

Le dispositif de mesure peut ainsi être placé sur le porteur :
- soit dans une première position, dans laquelle la première face est orientée vers le bras du porteur. Dans ce cas, ce sont les capteurs du premier jeu qui sont utilisés ;
- soit dans une deuxième position, dans laquelle la deuxième face est orientée vers le bras du porteur. Dans ce cas, ce sont les capteurs du deuxième jeu qui sont utilisés.

De façon concrète, le fait de prévoir que les capteurs de température cutanée du premier jeu s'étendent sur une longueur différente par rapport aux capteurs de température cutanée du deuxième jeu permet de disposer d'un dispositif de mesure polyvalent. Plus spécifiquement, le premier jeu peut être adapté aux bras ayant un périmètre dans une première gamme de valeurs de périmètres, et le deuxième jeu peut être adapté aux bras ayant un périmètre dans une deuxième gamme de valeurs de périmètres distincte de la première gamme. Un seul dispositif peut donc être prévu pour pouvoir être utilisé sur sensiblement n'importe quel porteur, quelle que soit sa morphologie ou sa corpulence.

Selon un deuxième aspect, l'invention concerne un ensemble comportant un dispositif de mesure tel que précédemment décrit et, en outre, un étui souple, destiné à recevoir le dispositif de mesure, l'étui possédant de préférence une fenêtre en regard de chacun des capteurs du dispositif de mesure, l'étui possédant, sur sa face destinée à être en contact avec le bras du porteur, au moins une portion adhésive destinée à être collée sur le bras du porteur.

La portion adhésive comprend par exemple une couche d'adhésif recouverte par au moins une bande pelable, avant la première utilisation.

Alternativement, le dispositif de mesure peut comprendre une portion de matériau adhérant non adhésif, par exemple en polyuréthane ou silicone basse densité, ladite portion étant destinée à être positionnée sur le bras du porteur. Cette portion a l'avantage d'être nettoyable et sa capacité d'adhérence est moins forte qu'un adhésif, ce qui permet de ne pas avoir de difficulté à enlever le dispositif de mesure du bras sur lequel il est positionné. L'adhérence de cette portion de matériau adhérant non adhésif étant généralement moins forte que celle d'un adhésif, cette portion stabilise le dispositif de mesure sur le bras mais doit être complétée par une bande d'adhésif rapportée, par exemple par une infirmière, pour relier de manière fiable les deux extrémités du dispositif de mesure.

Selon un troisième aspect, l'invention concerne un système de détermination d'une température interne profonde d'un être humain, qui comprend :
- un dispositif de mesure d'une pluralité de températures ou un ensemble tel que précédemment décrit ;
- une unité de traitement configurée et/ou programmée pour déterminer la température interne profonde du porteur du dispositif de mesure, à partir des données de température mesurées par ledit dispositif de mesure.

La valeur inférée peut être obtenue par un algorithme se référant à une table de correspondance construite par expérience.

Selon une caractéristique de l'invention, le dispositif de mesure comprend au moins un émetteur configuré pour transmettre à un récepteur les informations de mesure de température mesurées. L'émetteur peut être une antenne de transmission des informations de mesure de température.

Le dispositif de mesure peut comprendre un organe d'affichage de la température interne profonde déterminée, l'organe d'affichage pouvant être un écran indicateur et/ou un ou des indicateurs lumineux, par exemple des diodes électroluminescentes colorées ou non. Le ou les indicateurs lumineux permettent par exemple de renseigner un ou plusieurs états de fonctionnement du dispositif de mesure.

L'unité de traitement peut comprendre au moins une mémoire, qui est préférentiellement une mémoire cache permettant de collecter les données de température mesurées à des intervalles de temps réguliers ou non par le dispositif de mesure et de les conserver.

L'unité de traitement peut comprendre un récepteur configuré et/ou programmé pour coopérer avec un émetteur positionné sur le dispositif de mesure, par exemple une antenne de transmission. Le récepteur peut être configuré et/ou programmé pour communiquer à la mémoire de l'unité de traitement afin procéder au stockage des données de température reçues.

L'unité de traitement peut comprendre un dispositif d'affichage, configuré pour afficher au moins la température interne profonde déterminée par l'unité de traitement à partir des mesures réalisées par le dispositif de mesure. Le dispositif d'affichage peut être configuré pour afficher les données de température brutes et leurs intervalles de mesure. Le dispositif d'affichage du système peut être différent et distinct de l'organe d'affichage du dispositif de mesure.

Les communications peuvent être effectuées en filaire ou en non filaire, par exemple en Bluetooth.

L'unité de traitement est de préférence agencée et/ou programmée pour mettre en oeuvre l'étape d) et/ou e) et/ou g) et/ou h) et/ou i) décrite par la suite.

L'invention a également pour objet un procédé de détermination d'une température interne profonde d'un être humain, ledit procédé étant mis en oeuvre au moyen d'un système de détermination selon l'invention, le procédé comprenant les étapes suivantes :
a) mesure d'au moins une température cutanée à un temps t ou sur une période de temps p, par au moins trois capteurs de température cutanée du système de détermination selon l'invention,
b) mesure d'au moins une température cavitaire à un temps t ou sur une période de temps p, par au moins un capteur de température cavitaire du dispositif de mesure du système de mesure et de détermination selon l'invention,
c) le dispositif de mesure envoie les données de température mesurées aux étapes a), b), par le biais d'un émetteur, à un récepteur équipant une unité de traitement du système de mesure et de détermination selon l'invention,
d) le récepteur de l'unité de traitement reçoit les données de température mesurées et les transmet à une mémoire de l'unité de traitement qui stocke les données de température,
   - l'unité de traitement compare les données de température cutanée pour chaque capteur et détermine la température cutanée du porteur pour chaque capteur pour un temps t ou une période p en utilisant par exemple la moyenne des températures mesurées pour ce capteur,
   - l'unité de traitement compare pour le ou chaque capteur de température cavitaire et détermine la température cavitaire pour le ou chaque capteur pour un temps t ou une période p en utilisant par exemple la moyenne de températures mesurées pour chaque capteur,
e) à partir de la donnée de température retenue pour chaque capteur de température cutanée et pour le ou chaque capteur de température cavitaire, pour un temps t ou sur au moins une période p, l'unité de traitement détermine la température interne profonde du porteur par exemple en utilisant une table de correspondance ou/et en appliquant un modèle de détermination basé sur une forêt d'arbres décisionnels et/ou sur un réseau de neurones, utilisant les données de températures pour chaque capteur pour un temps t ou une période p, ou un jeu de données correspondant aux données de températures pour chaque capteur relevées pendant une période glissante d'observation P.

Le procédé peut comprendre une étape f) réalisée en parallèle des étapes a) et/ou b) et/ou à la suite des étapes a) et b) et consistant en la mesure d'au moins une température proximale à un temps t ou sur une période de temps p. Dans ce cas, l'étape d) sera de préférence complétée par le traitement des mesures de température proximale, dans le but d'obtenir une valeur unique pour le ou chaque capteur de température proximale. Ainsi, pour le ou chaque capteur de température proximale, l'unité de traitement compare les valeurs et détermine la température proximale pour ce capteur pour un temps t ou une période p en utilisant par exemple la moyenne des températures mesurées pour chaque capteur. Le traitement e) intègrera alors cette ou ces données en complément des autres valeurs de températures aux fins de déterminer la température interne profonde.

Selon une caractéristique de l'invention, la température interne profonde peut être déterminée par l'unité de traitement en fonction de paramètres complémentaires provenant d'un ou plusieurs capteurs de données physico-chimiques complémentaires.

Préférentiellement, la détermination de la température interne profonde peut être réalisée à partir des mesures réalisées dans les étapes a), b), f) et à partir de données caractérisant un éveil physiologique, lesdites données étant mesurées par un photoplethysmographe et/ou un galvanomètre.

Encore plus préférentiellement, la détermination de la température interne profonde peut être réalisée à partir des mesures réalisées dans les étapes a), b), f) et à partir de données caractérisant un éveil physiologique, et à partir de données de positionnement mesurées par un accéléromètre à trois axes par exemple.

Préférentiellement, la détermination de la température interne profonde peut être menée à partir des mesures réalisées pendant une période préférentiellement glissante P pendant laquelle on réalise une succession d'étapes a), d'étapes b), d'étapes f) et pendant laquelle on collecte un jeu de données caractérisant un éveil physiologique sur cette même période P.

Selon une caractéristique de l'invention, après que la température interne profonde du porteur a été déterminée pour un temps t ou sur au moins une période p, l'unité de traitement peut ordonner au dispositif d'affichage d'afficher la température interne profonde déterminée, au cours d'une étape g).

L'unité de traitement, à partir de la température interne profonde déterminée, peut être configurée et/ou programmée pour déterminer un état du porteur, au cours d'une étape h), par exemple en corrélant la température interne profonde mesurée à un état à partir d'une base de données ou un abaque intégrés dans la mémoire de l'unité de traitement. Par état du porteur on entend par exemple état sain, état fébrile, état critique.

Selon une caractéristique de l'invention, l'état du porteur peut être communiqué par l'unité de traitement au dispositif d'affichage qui affiche l'état du porteur, au cours d'une étape i).

Le procédé selon l'invention peut en outre comprendre les étapes suivantes :
- Après l'étape a), une étape a') de détermination d'une valeur unique de température cutanée, de préférence en choisissant la valeur la plus élevée parmi les températures cutanées des ou mesurées par les au moins trois capteurs cutanés,
- Après l'étape b), une étape b') de détermination d'une valeur unique de température cavitaire, de préférence en choisissant la valeur la plus élevée parmi les températures cavitaires de ou mesurées par l'au moins un capteur de température cavitaire.
- à partir de la valeur unique de température cutanée déterminée en étape a', de la valeur unique de température cavitaire déterminée en étape b', l'unité de traitement détermine la température interne profonde du porteur être humain.

Le procédé selon l'invention peut en outre comprendre les étapes suivantes :
- une étape FFF de mesure d'au moins une température proximale à un temps t ou sur une période de temps p, par l'au moins un capteur de température proximale,
- une étape FFF' de détermination d'une valeur unique de température proximale, de préférence en choisissant la valeur la plus élevée parmi les températures proximales de ou mesurées par l'au moins un capteur de température proximale
- à partir de la valeur unique de température cutanée déterminée en étape a', de la valeur unique de température cavitaire déterminée en étape b', de la valeur unique de température proximale déterminée en étape FFF', l'unité de traitement détermine la température interne profonde du porteur être humain.

### Brève description des figures

L'invention sera mieux comprise, grâce à la description ci-après, qui se rapporte à des modes de réalisation selon la présente invention, donnés à titre d'exemples non limitatifs et expliqués avec référence aux figures schématiques annexées. Les figures schématiques annexées sont listées ci-dessous :
[Fig. 1] est une vue en plan du dispositif de mesure selon un mode de réalisation de l'invention, en configuration non-portée, du côté de sa deuxième face ;
[Fig. 2] est une vue en plan du dispositif de mesure de la figure 1, en configuration non-portée, du côté de sa première face ;
[Fig. 3] est une vue similaire à la figure 1, montrant par transparence les composants intérieurs du dispositif de mesure ;
[Fig. 4] est une vue partielle de côté du dispositif de mesure de la figure 1, en configuration non-portée ;
[Fig. 5] est une vue en perspective éclatée d'une partie du dispositif de mesure de la figure 1, montrant une nappe allongée et un boîtier ;
[Fig. 6] est une vue en perspective de la nappe de la figure 5 ;
[Fig. 7a] est une vue en perspective d'un étui pour recevoir un dispositif de mesure selon un mode de réalisation l'invention, montrant une face de l'étui ;
[Fig. 7b] est une vue similaire à la figure 7a, montrant une face opposée de l'étui ;
[Fig. 8] est une vue en coupe du dispositif de mesure de la figure 1 en configuration portée ;
[Fig. 9] est une représentation schématique d'un système de mesure et de détermination selon un mode de réalisation de l'invention, le dispositif étant en configuration portée ;
[Fig. 10] est une vue partielle en perspective d'une variante de réalisation d'un dispositif selon l'invention, en configuration non-portée ;
[Fig. 11] est une vue partielle en perspective d'une autre variante de réalisation d'un dispositif selon l'invention, en configuration non-portée ;
[Fig. 12] est une vue partielle en perspective d'encore une autre variante de réalisation d'un dispositif selon l'invention, en configuration non-portée ;
[Fig. 13a] est une vue schématique de la première face d'un dispositif de mesure selon un autre mode de réalisation, en configuration non-portée ;
[Fig. 13b] est une vue schématique de la deuxième face du dispositif de mesure de la figure 13a, en configuration non-portée ;
[Fig. 13c] illustre le dispositif de mesure de la figure 13a en configuration portée, dans une première position ;
[Fig. 13d] illustre le dispositif de mesure de la figure 13a en configuration portée, dans une deuxième position.

Ces modes de réalisation étant nullement limitatifs, on pourra notamment considérer des variantes de l'invention ne comprenant qu'une sélection de caractéristiques décrites ou illustrées par la suite isolées des autres caractéristiques décrites ou illustrées (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection comprend au moins une caractéristique de préférence fonctionnelle sans détails structurels, et/ou avec seulement une partie des détails structurels si cette partie uniquement est suffisante pour conférer un avantage technique ou à différencier l'invention par rapport à l'état de la technique antérieure.

### Description détaillée

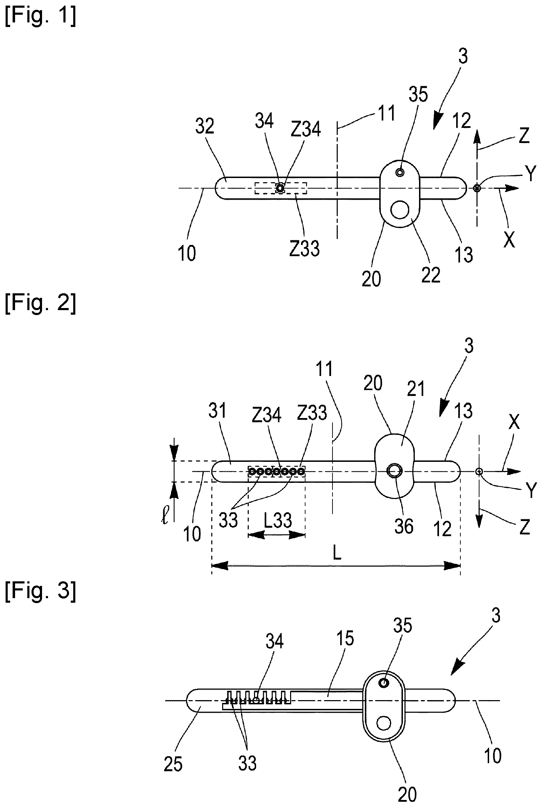

Les figures 1 à 6 représentent, certaines partiellement, un dispositif de mesure 3 d'une pluralité de températures selon un mode de réalisation de l'invention.

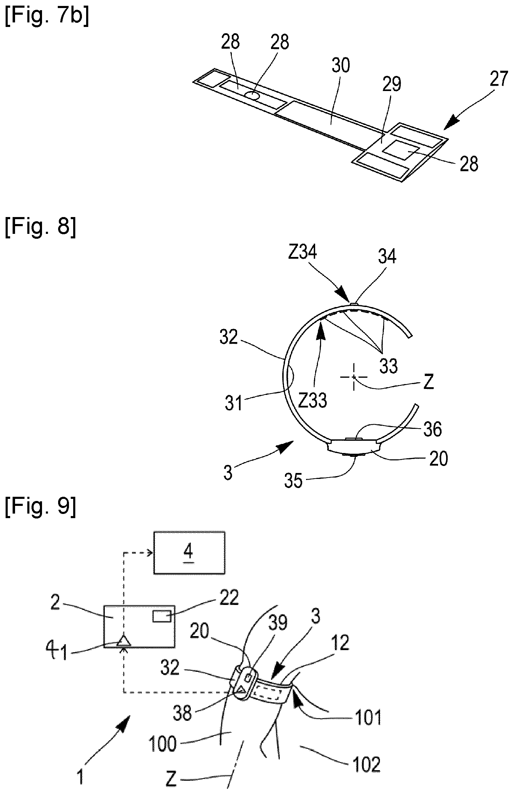

Sur les figures 1 à 4, le dispositif de mesure 3 est représenté dans sa configuration non portée dans laquelle, dans l'exemple non limitatif illustré, le dispositif de mesure 3 est sensiblement plan ; sur les figures 8 et 9, le dispositif de mesure 3 est représenté dans sa configuration portée dans laquelle il est au moins partiellement enroulé sur lui-même et forme au moins une portion de cylindre d'axe (Z).

En configuration portée, comme illustré sur la figure 9, le dispositif de mesure 3 entoure au moins partiellement, de manière transversale, un bras 100 d'un porteur 102, à proximité d'une aisselle 101 correspondante du corps du porteur, de sorte que l'axe (Z) du dispositif de mesure 3 soit sensiblement confondu avec l'axe dudit bras 100.

Dans la réalisation représentée, le dispositif de mesure 3 est un brassard entourant le bras d'un être humain, de façon totale ou partielle selon la circonférence du bras 100 et la taille du dispositif de mesure 3.

Le dispositif de mesure 3 comprend une première face 31 (visible sur la figure 2) et une deuxième face 32 (visible sur la figure 1) opposée à la première face 31.

En configuration portée, la première face 31 du dispositif de mesure 3 est tournée vers l'axe (Z) comme on le voit sur la figure 8, la première face 31 étant alors en contact avec la peau du bras 100 du porteur.

Comme illustré sur les figures 1 à 4, le dispositif de mesure 3 peut se présenter sous la forme d'une bande allongée dans une direction longitudinale (X) qui, dans la configuration portée, correspond sensiblement à une ligne périphérique du dispositif de mesure 3 et à une ligne périphérique autour du bras 100. Dans le mode de réalisation où le dispositif de mesure 3 est sensiblement plan en configuration non portée, les faces 31, 32 sont situées sensiblement dans des plans parallèles à (X, Z) en configuration non portée.

On définit l'axe (Y) comme étant orthogonal aux axes (X) et (Z).

Les trois axes (X) (Y) et (Z) sont orthogonaux entre eux.

La bande présente un axe longitudinal médian 10, un axe transversal médian 11, un bord supérieur 12 et un bord inférieur 13 (en référence à la position portée du dispositif de mesure 3, le porteur étant dans la position anatomique de référence). Il est précisé que les termes « bord supérieur » et « bord inférieur » sont utilisés à fins de simplification de la description, et employés ici en référence aux figures, notamment à la figure 9 dans laquelle le dispositif de mesure 3 est porté sur le bras droit du porteur. Toutefois, le même dispositif de mesure 3 pourrait être mis en place sur le bras gauche du porteur, avec une disposition inversée, le bord supérieur se trouvant alors en-dessous du bord inférieur.

Selon l'invention, on peut vouloir installer le dispositif de mesure sur le bras gauche du porteur. On peut avantageusement retourner le dispositif suivant l'axe antéro-postérieur du corps, si bien que le bord supérieur se trouve alors en-dessous du bord inférieur. L'avantage de cette option est que la manipulation de placement du dispositif sur le bras gauche est l'exacte symétrique à la manipulation de placement sur le bras droit par rapport au plan sagittal du corps, ce qui est un réel avantage ergonomique et cognitif pour les soignants, de plus tous les capteurs sont positionnés sur les emplacements du corps symétriques aux emplacements précédents, par rapport au plan sagittal du corps.

On peut alternativement passer le dispositif du bras droit au bras gauche sans le retourner, mais en ayant soin bien sûr de le tourner sur lui-même autour de l'axe Z, afin de positionner les capteurs cutanés centrés face à l'artère brachiale du bras gauche. Cette alternative est moins intéressante que l'alternative précédente pour la cohérence des résultats puisque les capteurs ne sont potentiellement pas positionnés sur des zones symétriques du corps selon le plan sagittal.

La bande présente une longueur L selon la direction (X), de préférence supérieure à 12 cm, encore mieux supérieure à 15 cm, voire supérieure à 20 cm, et une largeur I, selon une direction qui est sensiblement parallèle à l'axe (Z) du cylindre en configuration portée, d'environ 3 cm.

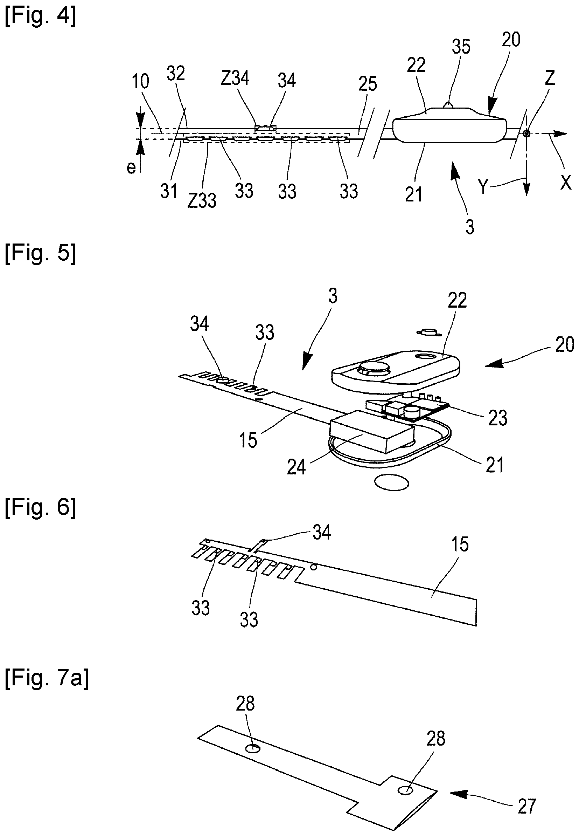

Comme on le voit sur les figures 3, 5 et 6, le dispositif de mesure 3 comprend une nappe 15 allongée selon la direction (X), et un boîtier 20 pouvant être formé d'une base 21 et d'un couvercle 22 pouvant être assemblé à la base 21. Le boîtier 20 contient une carte électronique 23 et une batterie 24 connectées à la nappe 15 allongée. La nappe 15 présente une épaisseur e, selon la direction (Y), comme illustré sur la figure 4.

Selon l'invention, le dispositif de mesure 3 comprend une pluralité de capteurs de température cutanée 33, plus précisément au moins trois capteurs de température cutanée 33, qui sont configurés pour mesurer une température cutanée du porteur 102. Les capteurs de température cutanée 33 sont positionnés sur ou au voisinage de la première face 31 du dispositif de mesure 3, et s'étendent sur au moins une partie d'une ligne périphérique du dispositif de mesure 3 en configuration portée.

Les au moins trois capteurs de température cutanée sont agencés pour être en contact direct de la peau du bras du porteur sans couche intermédiaire entre les au moins trois capteurs de température cutanée et la peau du porteur.

La première face du dispositif de mesure est agencée pour être en contact direct de la peau du bras du porteur sans couche intermédiaire entre la première face et la peau du bras du porteur, et les au moins trois capteurs de température cutanée sont situés sur cette première face en étant orientés vers l'extérieur du dispositif 3.

Dans l'exemple illustré sur la figure 2, le dispositif de mesure 3 comprend sept capteurs de température cutanée 33. Bien entendu, l'invention n'est pas limitée à cet exemple ni à un nombre de capteurs particulier. Néanmoins, plus les capteurs sont nombreux, plus la mesure de la température cutanée sera précise.

En outre, le dispositif de mesure 3 comprend au moins un capteur de température cavitaire 34 qui est configuré pour mesurer une température dans ou au voisinage de l'aisselle 101 du porteur 102. Le capteur de température cavitaire 34, qui ici est unique, sans que cela soit limitatif, est agencé sur ou au voisinage de la deuxième face 32 du dispositif de mesure 3. Le capteur de température cavitaire 34 est orienté vers l'extérieur, vers l'aisselle 101, et est donc impacté dans certaines positions du porteur par les conditions de température de l'aisselle 101. Prévoir plusieurs capteurs de température cavitaire 34 permet d'obtenir une mesure plus précise de la température cavitaire.

L'au moins un capteur de température cavitaire est agencé pour être en contact direct de l'aisselle du porteur sans couche intermédiaire entre l'au moins un capteur de température cavitaire et l'aisselle du porteur.

La deuxième face du dispositif de mesure est agencée pour être en contact direct de l'aisselle du porteur sans couche intermédiaire entre la deuxième face et l'aisselle du porteur, et l'au moins un capteur de température cavitaire est situé sur cette deuxième face en étant orienté vers l'extérieur du dispositif 3.

Le nombre de capteur(s) de température cavitaire (34) est inférieur au nombre de capteurs de température cutanée (33).

De façon concrète, la nappe 15 allongée forme un support pour les capteurs de température cutanée 33 et le capteur de température cavitaire 34, et intègre aussi des organes de connexion électronique entre ces capteurs et la carte électronique 23.

Sur la figure 6 on voit le capteur de température cavitaire 34 sur le même plan que les capteurs de température cutanée 33 mais sur une languette désaxée. Cette configuration permet de retourner la languette sur 180° et de situer ainsi le capteur de température cavitaire 34 sur la deuxième face 32 du dispositif de mesure 3, donc à l'opposé des capteurs de température cutanée 33.

Le dispositif de mesure 3 peut également comporter au moins un capteur de température proximale 35 configuré pour mesurer une température environnante à proximité immédiate du bras 100 du porteur 102. Le capteur de température proximale 35 est agencé sur ou au voisinage de la deuxième face 32 du dispositif de mesure 3. Prévoir plusieurs capteurs de température proximale 35 permet d'obtenir une mesure plus précise de la température environnante.

L'au moins un capteur de température proximale est situé sur la deuxième face en étant orienté vers l'extérieur du dispositif 3.

L'au moins un capteur de température proximale est agencé pour être en contact direct avec l'air environnant le dispositif 3 sans couche intermédiaire entre l'au moins un capteur de température proximale et cet air.

En configuration non-portée, une distance entre un des capteurs de température cutanée (33) (ou un point situé sur un des capteurs de température cutanée (33) ou le centre des capteurs de température cutanée) et l'au moins un capteur de température proximale (35) (ou un point situé sur un des capteurs de température proximale (35) ou le centre des capteurs de température proximale) est supérieure à 10 cm (et/ou inférieure à 20cm).

Le dispositif de mesure 3 peut également comporter au moins un capteur de données physico-chimiques complémentaires 36, tel qu'un photoplethysmographe, configuré pour mesurer la fréquence cardiaque et l'oxymétrie du porteur, qui peuvent être agencés sur ou au voisinage de la première face 31 du dispositif de mesure 3 (dans ce cas, l'au moins un capteur de données physico-chimiques est situé sur la première face en étant orienté vers l'extérieur du dispositif 3 et/ou est agencé pour être en contact direct de la peau du bras du porteur sans couche intermédiaire entre l'au moins un capteur de données physico-chimiques et la peau du porteur), ou sur ou au voisinage de la deuxième face 32 du dispositif de mesure 3 (dans ce cas, l'au moins un capteur de données physico-chimiques situé sur la deuxième face en étant orienté vers l'extérieur du dispositif 3 et/ou est agencé pour être en contact direct avec l'air environnant le dispositif 3 sans couche intermédiaire l'au moins un capteur de données physico-chimiques et cet air).

En pratique, le capteur de température proximale 35 et le capteur de données physico-chimiques complémentaires 36 peuvent être placés au niveau du boîtier 20, respectivement sur le couvercle 22 et sur la base 21.

Par ailleurs optionnellement, le dispositif de mesure 3 comprend les capteurs d'un galvanomètre 37 (comprenant deux capteurs) configuré pour mesurer le niveau de transpiration et de perspiration sécrété par le corps du porteur.

Le boîtier 20 et la nappe 15 allongée sont englobés, par exemple par surmoulage, dans une enveloppe 25 réalisée en une matière souple telle qu'un silicone.

Ainsi, le dispositif de mesure 3 est souple et configuré pour pouvoir être déformé de sa configuration non portée, par exemple plane, à sa configuration portée, cylindrique. Cette déformation se fait typiquement par enroulement, en courbant l'axe longitudinal médian 10 pour le faire passer d'une forme droite à une forme sensiblement circulaire (ou en variante : en portion de cercle, en hélice ou en spirale). Le dispositif de mesure 3 peut ainsi s'adapter à la morphologie du porteur et épouser la forme du bras 100.

Les capteurs de température cutanée 33 sont agencés dans une première zone Z33 du dispositif de mesure 3. Selon un mode de réalisation, la première zone Z33 est allongée et s'étend le long de la direction longitudinale (X) du dispositif de mesure 3. Dans la réalisation de la figure 1, les capteurs de température cutanée 33 sont disposés de façon alignée. Selon une variante non illustrée, les capteurs de température cutanée 33 pourraient être agencés en quinconce dans ladite première zone Z33.

À titre d'exemple, la longueur L33 de la première zone Z33 peut être de l'ordre de 5 cm.

Le ou les capteurs de température cavitaire 34 sont agencés dans une deuxième zone Z34 du dispositif de mesure 3.

La première zone Z33 des capteurs de température cutanée 33 et la deuxième zone Z34 du ou des capteurs de température cavitaire 34 peuvent être agencées totalement en regard l'une de l'autre, en projection dans un plan (X,Z), comme c'est le cas sur la figure 1. Comme on le voit sur la figure 1, le capteur de température cavitaire 34 peut être agencé de façon sensiblement centrée en regard de cette première zone Z33, selon la direction longitudinale X.

Selon une autre variante, la première zone Z33 des capteurs de température cutanée 33 et la deuxième zone Z34 du ou des capteurs de température cavitaire 34 peuvent être agencées seulement partiellement en regard l'une de l'autre, en projection dans un plan (X,Z), avec un certain recouvrement.

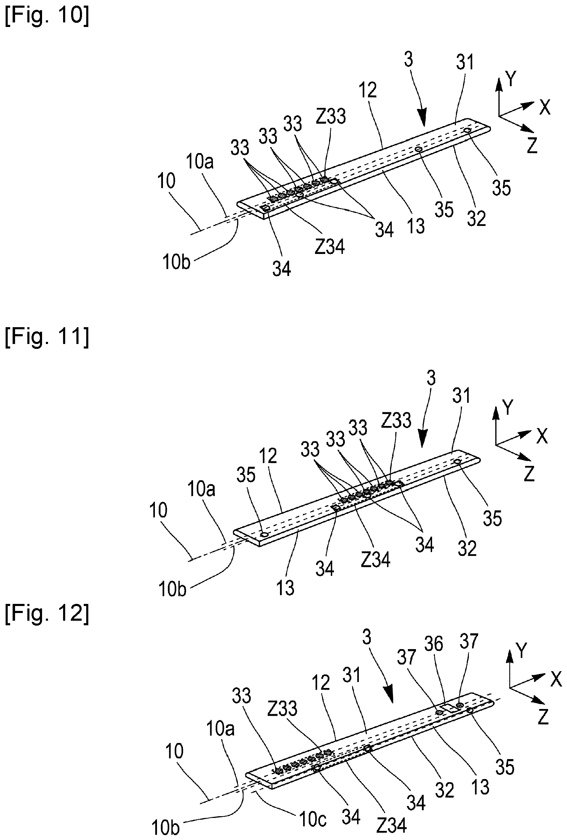

Selon encore une autre variante, la première zone Z33 des capteurs de température cutanée 33 et la deuxième zone Z34 du ou des capteurs de température cavitaire 34 peuvent être disjointes. Elles peuvent être de préférence sensiblement adjacentes en projection dans un plan (X,Z), comme c'est le cas sur la figure 12.

Le capteur de température proximale 35 est de préférence positionné de façon sensiblement diamétralement opposée au capteur de température cavitaire 34, en configuration portée du dispositif de mesure 3, comme on le voit sur la figure 8.

L'invention prévoit également un étui 27, illustré sur les figures 7a et 7b, pour recevoir le dispositif de mesure 3. Cet étui 27, qui est réalisé en une matière souple, permet de protéger le dispositif de mesure 3, de répondre à des considérations d'hygiène (l'étui pouvant être jetable ou bien lavable), et de faciliter la mise en place du dispositif de mesure 3 sur le porteur 102.

L'étui 27 possède de préférence une fenêtre 28 en regard de chacun des capteurs 33, 34, 35, 36, 37 du dispositif de mesure 3 qu'il reçoit, ou bien une fenêtre unique en regard du groupe de capteurs. En outre, l'étui peut posséder, sur sa face 29 destinée à être en contact avec le bras 100 du porteur 102, au moins une portion 30 adhésive destinée à être collée sur le bras 100 du porteur 102. Par exemple, la portion adhésive 30 comprend une couche d'adhésif recouverte par au moins une bande pelable, avant la mise en place du dispositif de mesure.

La figure 9 illustre un système 1 de détermination d'une température interne profonde d'un être humain. Le système 1 comprend le dispositif de mesure 3, ainsi qu'une unité de traitement 2 configurée et/ou programmée pour déterminer la température interne profonde du porteur 102 du dispositif de mesure 3, à partir des données de température mesurées par ledit dispositif de mesure 3.

L'unité de traitement 2 comprend par exemple un circuit électronique analogique et/ou numérique, et/ou une unité centrale d'un ordinateur, et/ou un microprocesseur, et/ou des moyens logiciels.

L'unité de traitement 2 et le dispositif de mesure 3 sont configurés et/ou programmés pour communiquer l'un avec l'autre et en particulier, le dispositif de mesure 3 est configuré pour transmettre les données de températures mesurées à l'unité de traitement 2 qui les reçoit et les analyse pour déterminer à partir de ces données la température interne profonde du porteur du dispositif de mesure 3.

Pour communiquer avec l'unité de traitement 2, le dispositif de mesure 3 comprend par exemple un émetteur 38 configuré pour transmettre les informations de mesure de température mesurées à un récepteur 41 de l'unité de traitement 2, comme représenté en figure 9. L'émetteur 38 peut être une antenne de transmission des informations de mesure de température.

Par ailleurs, le dispositif de mesure 3 peut comprendre un organe d'affichage 39 de la température interne profonde déterminée, l'organe d'affichage 39 pouvant être un écran indicateur et/ou un ou des indicateurs lumineux, par exemple des diodes électroluminescentes colorées ou non. La température interne profonde déterminée par l'unité de traitement 2 est communiquée au dispositif de mesure 3 et particulièrement à l'organe d'affichage 39 par l'émetteur 38, qui est un émetteur/récepteur.

De façon concrète, l'émetteur 38 et l'organe d'affichage 39 peuvent être agencés sur ou dans le boîtier 20 du dispositif de mesure 3.

Selon un mode de réalisation, l'unité de traitement 2 comprend au moins une mémoire 22, ladite mémoire 22 étant préférentiellement une mémoire cache permettant de collecter les données de température mesurées à des intervalles de temps réguliers ou non par le dispositif de mesure et de les conserver. En outre, l'unité de traitement 2 peut comprendre un récepteur 41 configuré pour coopérer avec un émetteur 38 positionné sur le dispositif de mesure 3, par exemple une antenne de transmission. Le récepteur 41 est de préférence également émetteur et configuré pour communiquer avec la mémoire 22 de l'unité de traitement 2 afin de stocker les données de température reçues.

Le système 1 selon l'invention peut en outre comprendre un dispositif d'affichage 4 configuré pour afficher au moins la température interne profonde déterminée par l'unité de traitement 2 à partir des mesures réalisées par le dispositif de mesure 3.

Dans une version très intégrée de l'électronique, on peut prévoir que l'unité de traitement 2 soit intégrée dans le dispositif de mesure 3.

Différents agencements des capteurs sont illustrés sur les figures 10 à 12.

Un premier agencement est représenté sur la figure 10.

Selon cet agencement, les capteurs de température cutanée 33 sont alignés sur un axe 10a correspondant à la projection de l'axe longitudinal médian 10 du dispositif de mesure 3, le long de l'axe (Y), sur la première face 31.

En outre, peuvent être prévus plusieurs capteurs de température cavitaire 34 (par exemple trois) qui sont en regard de la zone Z33 dans laquelle se trouvent les capteurs de température cutanée 33. Les capteurs de température cavitaire 34, représentés en pointillés, peuvent être alignés sur un axe 10b correspondant à la projection de l'axe longitudinal médian 10 du dispositif de mesure 3, le long de l'axe (Y), sur la deuxième face 32.

Vues en projection selon l'axe (Y), dans un plan de projection parallèle à (X,Z), les zones Z33 et Z34 sont ainsi au moins partiellement en regard l'une de l'autre, c'est-à-dire au moins partiellement superposées. La superposition peut ne pas être totale. Par exemple, comme on le voit sur la figure 10, la zone Z34 s'étend au-delà de la zone Z33 de long de l'axe (X), et ce de part et d'autre de la zone Z33. Une disposition inverse - c'est-à-dire dans laquelle la zone Z33 s'étendrait au-delà de la zone Z34 de long de l'axe (X) - est également envisageable.

Par exemple, vu dans ledit plan de projection, et le long de l'axe (X), on peut avoir un premier capteur de température cavitaire 34 positionné de façon centrée par rapport à la première zone Z33, et deux capteurs de température cavitaire 34 positionnés de part et d'autre et à proximité immédiate des extrémités de la première zone Z33.

En outre, les capteurs de température proximale 35 - par exemple au nombre de deux - peuvent être alignés sur l'axe 10b précité. Les capteurs de température proximale sont par exemple disposés à une partie extrême du dispositif de mesure 3, selon l'axe (X), tandis que les zones Z33 et Z34 sont disposées à l'autre partie extrême du dispositif de mesure 3, selon l'axe (X). Bien entendu, l'invention n'est pas limitée à cette disposition.

Dans cette « configuration portée » du dispositif de mesure (posé sur le bras de l'utilisateur), comportant des capteurs de température proximale localisés en deux sous-groupes aux deux extrémités du dispositif de mesure, les deux sous-groupes se rapprochent ou se rassemblent ou se superposent ne formant plus qu'un seul groupe.

Un autre agencement est illustré sur la figure 11.

Selon cet agencement, le groupe formé par les zones Z33, Z34, et les capteurs 33, 34 est identique à ce qui est décrit ci-dessus en référence à la figure 10. En revanche, dans la réalisation de la figure 11, ce groupe est situé de façon sensiblement centrée sur le dispositif de mesure 3, le long de l'axe (X).

Un capteur de température proximale 35 est prévu à chaque extrémité du dispositif de mesure 3. Les deux capteurs de température proximale 35 sont également situés sur l'axe 10b précité. Leur localisation aux deux extrémités du dispositif de mesure 3 en configuration non portée, fait qu'en configuration portée, les deux capteurs de température proximale 35 se positionnent tous deux de façon sensiblement diamétralement opposée au centre de la zone Z33, pour autant que le dispositif de mesure 3 soit mis en place sur un bras dont le diamètre fait partie de la gamme pour laquelle le dispositif de mesure 3 a été prévu et dimensionné.

Un troisième agencement est illustré sur la figure 12.

Dans cet agencement, les capteurs de température cutanée 33 sont agencés comme sur la figure 10. Sont également prévus, sur la première face 31, et sensiblement sur l'axe 10a, un capteur de données physico-chimiques complémentaires 36 et au moins les deux capteurs 37 d'au moins un galvanomètre, agencés de part et d'autre du capteur de données physico-chimiques complémentaires 36. Le groupe des capteurs 36, 37 peut être agencé au voisinage de l'extrémité du dispositif de mesure 3 opposée à l'extrémité où est située la zone Z33, selon l'axe (X).

Un ou plusieurs capteurs de température cavitaire 34, ici au nombre de deux, sont également prévus. Dans la réalisation de la figure 12, les capteurs de température cavitaire 34 sont alignés sur un axe 10c qui est situé sur la deuxième face 32, et qui est parallèle à l'axe 10b en étant décalé de celui-ci selon l'axe (Z). L'axe 10c peut être décalé de l'axe 10b en direction du bord inférieur 13.

Vues en projection selon l'axe (Y), dans un plan de projection parallèle à (X,Z), les zones Z33 et Z34 sont ainsi décalées l'une de l'autre selon la direction (Z). Selon les dimensions des capteurs 33, 34 et le décalage entre les axes 10b et 10c, il peut y avoir un certain recouvrement entre les zones Z33 et Z34 selon l'axe (Z), ou bien les zones Z33 et Z34 peuvent être adjacentes selon l'axe (Z), ou encore les zones Z33 et Z34 peuvent être espacées l'une de l'autre selon l'axe (Z). En outre, dans la réalisation représentée sur la figure 12, la deuxième zone Z34 est également décalée par rapport à la première zone Z33 le long de l'axe (X). Par exemple, vu dans ledit plan de projection, et en considérant uniquement le positionnement relatif en projection sur l'axe (X), on peut avoir un premier capteur de température cavitaire 34 positionné au niveau de la première zone Z33, pas forcément de façon centrée contrairement à l'agencement de la figure 10, et un second capteur de température cavitaire 34 positionné à distance de la zone Z33, en direction des capteurs 36, 37. Cette disposition n'est pas limitative.

Le dispositif de mesure 3 peut également comporter un ou plusieurs capteurs de température proximale 35. Dans la réalisation représentée, il est prévu un unique capteur de température proximale 35, disposé sur l'axe 10c, à la partie extrême du dispositif de mesure 3 opposée à la zone Z33, selon l'axe (X).

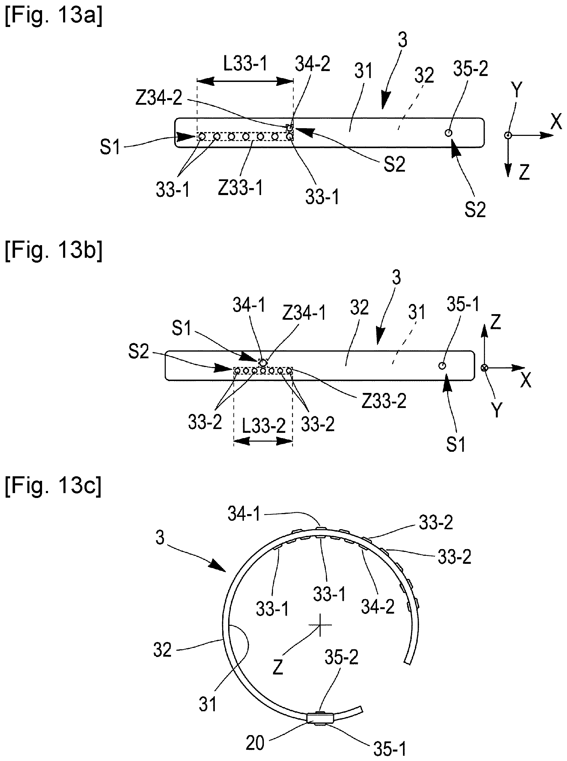

On se réfère à présent aux figures 13a à 13d qui illustrent un autre mode de réalisation de l'invention. Selon ce mode de réalisation, on prévoit de cumuler sur un même dispositif de mesure deux jeux complets de capteurs pour deux positions différentes sur le bras du porteur. Le choix de la position est établi au moment où l'on installe le dispositif de mesure sur le bras du porteur, selon que l'on met une face ou bien l'autre en contact avec la peau.

Comme illustré schématiquement sur les figures 13a et 13b, le dispositif de mesure 3 comprend un premier jeu de capteurs S1 et un deuxième jeu de capteurs S2.

Le premier jeu de capteurs S1 comprend :
- au moins trois capteurs de température cutanée 33-1 qui sont positionnés sur ou au voisinage de la première face 31 du dispositif de mesure dans une première zone Z33-1, et qui s'étendent sur une première longueur L33-1 ; les au moins trois capteurs de température cutanée du premier jeu sont agencés pour être en contact direct de la peau du bras du porteur sans couche intermédiaire entre les au moins trois capteurs de température cutanée du premier jeu et la peau du porteur, et/ou la première face du dispositif de mesure est agencée pour être en contact direct de la peau du bras du porteur sans couche intermédiaire entre la première face et la peau du bras du porteur, et les au moins trois capteurs de température cutanée du premier jeu sont situés sur cette première face en étant orientés vers l'extérieur du dispositif 3 ;
- au moins un capteur de température cavitaire 34-1 qui est agencé sur ou au voisinage de la deuxième face 32 du dispositif de mesure 3 dans une deuxième zone Z34-1 ; l'au moins un capteur de température cavitaire du premier jeu est agencé pour être en contact direct de l'aisselle du porteur sans couche intermédiaire entre l'au moins un capteur de température cavitaire du premier jeu et l'aisselle du porteur, et/ou la deuxième face du dispositif de mesure est agencée pour être en contact direct de l'aisselle du porteur sans couche intermédiaire entre la deuxième face et l'aisselle du porteur, et l'au moins un capteur de température cavitaire du premier jeu est situé sur cette deuxième face en étant orienté vers l'extérieur du dispositif 3 ;
- et, de préférence, un capteur de température proximale 35-1 qui est agencé sur ou au voisinage de la deuxième face 32 du dispositif de mesure 3.

En outre, comme déjà décrit, les zones Z33-1 et Z34-1 sont au moins en partie en regard l'une de l'autre ou sont sensiblement adjacentes, en projection dans un plan orthogonalement aux première et deuxième faces 31, 32.

Le deuxième jeu de capteurs S2 comprend :
- au moins trois capteurs de température cutanée 33-2 qui sont positionnés sur ou au voisinage de la deuxième face 32 du dispositif de mesure 3, dans une première zone Z33-2, et qui s'étendent sur une deuxième longueur L33-2 avantageusement différente de la première longueur L33-1. Dans la réalisation représentée, L33-2 est inférieure à L33-1. En outre, on peut avoir un espacement différent entre capteurs de température cutanée adjacents selon que ces capteurs appartiennent au premier jeu S1 ou au deuxième jeu S2 ; les au moins trois capteurs de température cutanée du deuxième jeu sont agencés pour être en contact direct de la peau du bras du porteur sans couche intermédiaire entre les au moins trois capteurs de température cutanée du deuxième jeu et la peau du porteur, et/ou la deuxième face du dispositif de mesure est agencée pour être en contact direct de la peau du bras du porteur sans couche intermédiaire entre la deuxième face et la peau du bras du porteur, et les au moins trois capteurs de température cutanée du deuxième jeu sont situés sur cette deuxième face en étant orientés vers l'extérieur du dispositif 3 ;
- au moins un capteur de température cavitaire 34-2 qui est agencé sur ou au voisinage de la première face 31 du dispositif de mesure 3, dans une deuxième zone Z34-2 ; l'au moins un capteur de température cavitaire du deuxième jeu est agencé pour être en contact direct de l'aisselle du porteur sans couche intermédiaire entre l'au moins un capteur de température cavitaire du deuxième jeu et l'aisselle du porteur, et/ou la première face du dispositif de mesure est agencée pour être en contact direct de l'aisselle du porteur sans couche intermédiaire entre la première face et l'aisselle du porteur, et l'au moins un capteur de température cavitaire du deuxième jeu est situé sur cette première face en étant orienté vers l'extérieur du dispositif 3 ;
- et, de préférence, un capteur de température proximale 35-2 qui est agencé sur ou au voisinage de la première face 31 du dispositif de mesure 3.

Le dispositif est agencé pour utiliser ou activer un seul jeu de capteurs à la fois parmi le premier jeu de capteurs et le deuxième jeu de capteurs : un seul jeu de capteurs à la fois parmi le premier jeu de capteurs et le deuxième jeu de capteurs est activé pour mesurer au moins une température, tandis que l'autre jeu de capteurs parmi le premier jeu de capteurs et le deuxième jeu de capteurs est inactif et n'est pas utilisé pour mesurer une ou plusieurs températures.

Le dispositif 3 et/ou le système 1 (plus précisément les moyens électroniques et/ou logiciels 23 et/ou l'unité de traitement 2) est agencé et/ou programmé pour déterminer quelle face respectivement 31 ou 32 est orientée vers le bras de l'utilisateur et quelle face respectivement 32 ou 31 est orientée vers l'aisselle de l'utilisateur, et :
- si la face 31 est orientée vers le bras de l'utilisateur et la face 32 est orientée vers l'aisselle de l'utilisateur, activer le premier jeu de capteurs et désactiver le deuxième jeu de capteurs, et
- si la face 32 est orientée vers le bras de l'utilisateur et la face 31 est orientée vers l'aisselle de l'utilisateur, activer le deuxième jeu de capteurs et désactiver le premier jeu de capteurs.

Le dispositif 3 et/ou le système 1 (plus précisément les moyens électroniques et/ou logiciels 23 et/ou l'unité de traitement 2) est agencé et/ou programmé pour assigner la face orientée vers l'aisselle de l'utilisateur comme étant la face, parmi les faces 31 et 32 :
- ayant des variations de température mesurées (par les capteurs 33-1, 33-2, 34-1 et/ou 34-2) de plus fortes amplitudes et/ou de fréquence temporelle plus élevée (car dans son utilisation normale, le bras se sépare régulièrement de l'aisselle alors que le dispositif 3 reste fixé au bras), et/ou
- ayant une température mesurée (par les capteurs 33-1, 33-2, 34-1 et/ou 34-2) la plus élevée.

Ainsi, dans le procédé selon l'invention mis en oeuvre dans le dispositif 3 et/ou système 1 (plus précisément par les moyens électroniques et/ou logiciels 23 et/ou l'unité de traitement 2), on a une détermination de quelle face respectivement 31 ou 32 est orientée vers le bras de l'utilisateur et quelle face respectivement 32 ou 31 est orientée vers l'aisselle de l'utilisateur, et :
- si la face 31 est orientée vers le bras de l'utilisateur et la face 32 est orientée vers l'aisselle de l'utilisateur, une activation du premier jeu de capteurs et une désactivation du deuxième jeu de capteurs, et
- si la face 32 est orientée vers le bras de l'utilisateur et la face 31 est orientée vers l'aisselle de l'utilisateur, une activation du deuxième jeu de capteurs et une désactivation du premier jeu de capteurs.
   avec de préférence une assignation de la face orientée vers l'aisselle de l'utilisateur comme étant la face, parmi les faces 31 et 32 :
   - ayant des variations de température mesurées (par les capteurs 33-1, 33-2, 34-1 et/ou 34-2) de plus fortes amplitudes et/ou de fréquence temporelle plus élevée (car dans son utilisation normale, le bras se sépare régulièrement de l'aisselle alors que le dispositif 3 reste fixé au bras), et/ou
   - ayant une température mesurée (par les capteurs 33-1, 33-2, 34-1 et/ou 34-2) la plus élevée.

Comme illustré sur les figures, le nombre de capteur(s) de température cavitaire (34-1) du premier jeu est inférieur au nombre de capteurs de température cutanée (33-1) du premier jeu, et
Comme illustré sur les figures, le nombre de capteur(s) de température cavitaire (34-2) du deuxième jeu est inférieur au nombre de capteurs de température cutanée (33-2) du deuxième jeu.

En outre, comme déjà décrit, les zones Z33-2 et Z34-2 sont au moins en partie en regard l'une de l'autre ou sont sensiblement adjacentes, en projection dans un plan orthogonalement aux première et deuxième faces 31, 32.

Il est à noter que, sur la figure 13a, les zones Z34-2 et Z33-1 sont décalées l'une par rapport à l'autre selon la direction Z, et ce pour des raisons de clarté du dessin. Toutefois, cet agencement ne doit pas être considéré comme étant limitatif, les zones Z34-2 et Z33-1 pouvant être agencées de façon au moins partiellement superposée. La même remarque concerne la figure 13b. En outre, le nombre de capteurs dans une zone donnée, leur écartement relatif et la disposition relative des différentes zones de capteurs sont illustrés à titre d'exemple non limitatif.

Avantageusement, on peut superposer l'axe de la zone Z33-1 et l'axe de la zone Z34-2 afin d'avoir un ou plusieurs capteurs de température communs aux deux zones. De même peut avantageusement superposer l'axe de la zone Z33-2 et l'axe de la zone Z34-1 afin d'avoir un ou plusieurs capteurs de température commun aux deux zones. Ces configurations sont équivalentes en termes de précision des valeurs obtenues, mais présentent un réel avantage en terme économique puisque la même information est captée tout en nécessitant moins de capteurs.

Par ailleurs, bien que les capteurs 36, 37 ne soient pas représentés, cela ne signifie pas nécessairement qu'ils sont absents.

En d'autres termes, dans le dispositif de mesure 3 des figures 13a à 13d, On a cumulé deux implantations de capteurs de températures. Ceci permet, avec un même dispositif de mesure 3, de s'adapter à une large gamme de diamètres de bras.

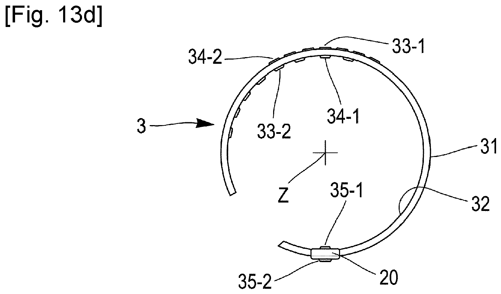

Le dispositif de mesure 3 illustré en configuration non portée plane sur les figures 13a et 13b peut être enroulé vers sa configuration portée, selon deux positions différentes. Dans une première position, illustré sur la figure 13c, la première face 31 est orientée vers le bras du porteur. Dans ce cas, ce sont les capteurs du premier jeu S1 qui sont utilisés. Dans une deuxième position, illustré sur la figure 13d, la deuxième face 32 est orientée vers le bras du porteur. Dans ce cas, ce sont les capteurs du deuxième jeu S2 qui sont utilisés.

Ainsi, le dispositif de mesure 3, dans la première position de la figure 13c, peut par exemple être destiné aux bras de circonférence comprise entre 20 cm et 28 cm ; et le même dispositif de mesure 3 peut être retourné dans la deuxième position illustrée sur la Figure 13d et équiper avantageusement un bras dont la circonférence est de 28 cm à 40 cm. L'agencement des capteurs dans chacun des jeux S1, S2 est configuré pour être adapté à ces deux gammes morphologiques.

Ainsi, lorsqu'un soignant est face à un patient dont le bras présente un périmètre inclus dans une première gamme de valeurs de périmètres, il choisira de poser la première face 31 côté peau ; lorsque le soignant est face à un patient dont le bras présente un périmètre inclus dans une deuxième gamme de valeurs de périmètres, il choisira de poser la deuxième face 32 côté peau. Dans les deux positions, le dispositif de mesure 3 est tout à fait adapté au porteur et fournit des mesures avec la précision requise.

Bien sûr on peut optimiser le nombre de capteurs et utiliser les capteurs de température implantés sur une face pour la première gamme de périmètres, pour réaliser les mesures nécessaires pour la seconde gamme de périmètres.

On note que ces deux jeux de capteurs S1, S2 coexistent sur le dispositif de mesure 3 et sont indépendantes. On peut par exemple prévoir un dispositif dont une face serait pour placer en haut de l'aisselle et une face serait pour placer un peu plus bas, ou un dispositif dont une face serait pour placer spécifiquement sous l'aisselle gauche et l'autre spécifiquement sous l'aisselle droite, etc.

La mesure de la température cutanée doit être très précise et très fiable. On a ainsi privilégié des capteurs de température 33 spécialisés pour la santé humaine, intégrant un convertisseur analogique-numérique haute résolution.

L'exigence de précision sur les mesures de température cavitaire est moins forte que pour les mesures de températures cutanées. On peut donc choisir des capteurs 34 plus simples, tels que des thermocouples ou des thermistances, qui sont plus simples à mettre en oeuvre et moins coûteuses.

Pour plus de simplicité, les capteur 33, 34 et 35 sont identiques : dans la présente description, chaque capteur de température, notamment de référence 33, 34, 35, est un capteur de température de référence « MAX30205 Human Body Température Sensor » fabriqué par la société MAXIM.

Un mode de réalisation de procédé selon l'invention de détermination d'une température interne profonde d'un être humain, mis en oeuvre dans un système 1 tel que précédemment décrit comprenant n'importe quelle variante de dispositif 3 tel que précédemment décrit, comprend les étapes suivantes :
a) mesure d'au moins une température cutanée à un temps t ou sur une période de temps p, par l'au moins trois capteurs de température cutanée 33 du système de détermination selon l'invention,
b) mesure d'au moins une température cavitaire à un temps t ou sur une période de temps p, par l'au moins un capteur de température cavitaire 34 du dispositif de mesure du système de mesure et de détermination selon l'invention,
c) le dispositif de mesure 3 envoie les données de température mesurées aux étapes a), b), par le biais de l'émetteur 38, au récepteur 41 équipant l'unité de traitement 2 du système de mesure et de détermination selon l'invention,
d) le récepteur 41 de l'unité de traitement reçoit les données de température mesurées et les transmet à la mémoire 22 de l'unité de traitement qui stocke les données de température,
   - l'unité de traitement 2 compare les données de température cutanée pour chaque capteur 33 et détermine la température cutanée du porteur pour chaque capteur 33 pour un temps t ou une période p en utilisant par exemple la moyenne des températures mesurées pour ce capteur 33,
   - l'unité de traitement 2 compare pour le ou chaque capteur 34 de température cavitaire et détermine la température cavitaire pour le ou chaque capteur 34 pour un temps t ou une période p en utilisant par exemple la moyenne de températures mesurées pour chaque capteur 34,
e) à partir de la donnée de température retenue pour chaque capteur de température cutanée 33 et pour le ou chaque capteur 34 de température cavitaire, pour un temps t ou sur au moins une période p, l'unité de traitement 2 détermine la température interne profonde du porteur par exemple en utilisant une table de correspondance ou/et en appliquant un modèle de détermination basé sur une forêt d'arbres décisionnels et/ou sur un réseau de neurones, utilisant les données de températures pour chaque capteur pour un temps t ou une période p, ou un jeu de données correspondant aux données de températures pour chaque capteur relevées pendant une période glissante d'observation P.

Le procédé peut comprendre une étape f) réalisée en parallèle des étapes a) et/ou b) et/ou à la suite des étapes a) et b) et consistant en la mesure d'au moins une température proximale à un temps t ou sur une période de temps p. Dans ce cas, l'étape d) sera complétée par le traitement des mesures de température proximale, dans le but d'obtenir une valeur unique pour le ou chaque capteur de température proximale 35. Ainsi, pour le ou chaque capteur de température proximale 35, l'unité de traitement 2 compare les valeurs et détermine la température proximale pour ce capteur 35 pour un temps t ou une période p en utilisant par exemple la moyenne des températures mesurées pour chaque capteur 35. Le traitement e) intègrera alors cette ou ces données en complément des autres valeurs de températures aux fins de déterminer la température interne profonde.

Un mode de réalisation de procédé selon l'invention de détermination d'une température interne profonde d'un être humain, mis en oeuvre dans un système 1 tel que précédemment décrit comprenant n'importe quelle variante de dispositif 3 tel que précédemment décrit, comprend les étapes suivantes :
L'étape a) précédemment décrite.

Une étape a' de détermination d'une valeur unique de température cutanée, notamment en choisissant la valeur la plus élevée parmi les températures cutanées des ou mesurées par les au moins 3 capteurs cutanés 33 du système de détermination selon l'invention, l'étape a' étant réalisée par le dispositif de mesure 3 ou l'unité de traitement 2.

L'étape b telle que précédemment décrite.

Une étape b' de détermination d'une valeur unique de température cavitaire, notamment en choisissant la valeur la plus élevée parmi les températures cavitaires de ou mesurées par l'au moins un capteur de température cavitaire 34 du dispositif de mesure du système de mesure et de détermination selon l'invention, l'étape b' étant réalisée par le dispositif de mesure 3 ou l'unité de traitement 2.

Une étape FFF, mesure d'au moins une température proximale à un temps t ou sur une période de temps p, par l'au moins un capteur de température proximale 35 du dispositif de mesure du système de mesure et de détermination selon l'invention.

Une étape FFF' de détermination d'une valeur unique de température proximale, notamment en choisissant la valeur la plus élevée parmi les températures proximales de ou mesurées par l'au moins un capteur de température proximale 35 du dispositif de mesure du système de mesure et de détermination selon l'invention, l'étape FFF' étant réalisée par le dispositif de mesure 3 ou l'unité de traitement 2.

À partir de la valeur unique de température cutanée déterminée en étape a', de la valeur unique de température cavitaire déterminée en étape b', de la valeur unique de température proximale déterminée en étape FFF', l'unité de traitement 2 détermine la température interne profonde du porteur par exemple en utilisant une table de correspondance ou/et en appliquant un modèle de détermination basé sur une forêt d'arbres décisionnels et/ou sur un réseau de neurones, utilisant la donnée unique de température cutanée, température cavitaire et température proximale retenue pour un temps t ou une période p, ou un jeu de données correspondant aux données de température cutanée, température cavitaire et température proximale pendant une période glissante d'observation P.

La température interne profonde peut être déterminée par l'unité de traitement 2 en fonction de paramètres complémentaires provenant d'un ou plusieurs capteurs de données physico-chimiques complémentaires tels que précédemment décrit(s).

Préférentiellement, la détermination de la température interne profonde peut être réalisée à partir des mesures réalisées dans les étapes a), b), f) et à partir de données caractérisant un éveil physiologique, lesdites données étant mesurées par un photoplethysmographe et/ou un galvanomètre.

Encore plus préférentiellement, la détermination de la température interne profonde peut être réalisée à partir des mesures réalisées dans les étapes a), b), f) et à partir de données caractérisant un éveil physiologique, et à partir de données de positionnement mesurées par un accéléromètre à trois axes par exemple.

Préférentiellement, la détermination de la température interne profonde peut être menée à partir des mesures réalisées pendant une période préférentiellement glissante P pendant laquelle on réalise une succession d'étapes a), d'étapes b), d'étapes f) et pendant laquelle on collecte un jeu de données caractérisant un éveil physiologique sur cette même période P.

Selon une caractéristique de l'invention, après que la température interne profonde du porteur a été déterminée pour un temps t ou sur au moins une période p, l'unité de traitement 2 peut ordonner au dispositif d'affichage 4 d'afficher la température interne profonde déterminée, au cours d'une étape g).

L'unité de traitement 2, à partir de la température interne profonde déterminée, peut être configurée et/ou programmée pour déterminer un état du porteur, au cours d'une étape h), par exemple en corrélant la température interne profonde mesurée à un état à partir d'une base de données ou un abaque intégrés dans la mémoire de l'unité de traitement 2. Par état du porteur on entend par exemple état sain, état fébrile, état critique.

Selon une caractéristique de l'invention, l'état du porteur peut être communiqué par l'unité de traitement 2 au dispositif d'affichage 4 qui affiche l'état du porteur, au cours d'une étape i).

Lors de chaque étape de mesure, le dispositif 3 est de préférence porté par l'utilisateur 102 dans sa configuration portée, en entourant au moins partiellement un bras 100 du porteur 102 :
- de préférence à proximité d'une aisselle 101 correspondante du corps du porteur 102, et/ou
- de préférence de sorte que l'axe Z du dispositif de mesure 3 soit sensiblement confondu avec l'axe dudit bras 100, et/ou
- de préférence la première face 31 du dispositif de mesure 3 étant alors en contact avec la peau du bras 100 du porteur 102.

La valeur de p est de préférence la même pour les capteurs 33 et/ou 34 et/ou 35.

La valeur de P est différente de la valeur de p.

La valeur de P est supérieure à la valeur de p.

La valeur de P est de préférence au moins trois fois supérieure à la valeur de p, de préférence au moins cinq fois supérieure à la valeur de p, de préférence au moins dix fois supérieure à la valeur de p.

La valeur de p est de préférence supérieure ou égale à 1 seconde, de préférence supérieure ou égale à 5 secondes, de préférence supérieure ou égale à 10 secondes.

La valeur de P est de préférence supérieure ou égale à 30 secondes, de préférence supérieure ou égale à 1 minute.

Typiquement, la fréquence d'acquisition choisie est de 1 Hz pour l'ensemble des capteurs de température. Pour chaque capteur de température on extrait une valeur toutes les 30 secondes, donc p = 30". La période glissante P choisie est de 5 minutes, autrement dit, on utilise 10 périodes p, donc un historique de 10 valeurs pour chaque capteur, aux fins d'inférer la valeur de la température centrale.

On peut ajouter à l'algorithme d'inférence un algorithme prédictif qui a pour objectif de prédire la valeur de la température pour les prochaines minutes, pour indiquer au soignant la tendance générale observée afin de l'aider à anticiper sur les actions à mener.

Bien entendu, l'invention n'est pas limitée aux modes de réalisation décrits et représentés aux figures annexées. Des modifications restent possibles, notamment du point de vue de la constitution des divers éléments, sans sortir pour autant du domaine de protection de l'invention, tel que défini par les revendications.

## Revendications

1. Dispositif de mesure d'une pluralité de températures en vue de la détermination d'une température interne profonde d'un être humain (102), porteur dudit dispositif de mesure, le dispositif de mesure (3) présentant une première face (31) et une deuxième face (32) opposée à la première face (31) et étant configuré pour pouvoir être dans une configuration portée dans laquelle le dispositif de mesure (3) est au moins partiellement enroulé sur lui-même et forme au moins une portion de cylindre d'axe (Z), la première face (31) étant tournée vers l'axe (Z), le dispositif de mesure (3), dans sa configuration portée, étant destiné à entourer au moins partiellement un bras (100) du porteur (102), à proximité d'une aisselle (101) correspondante du corps du porteur (102), de sorte que l'axe (Z) du dispositif de mesure (3) soit sensiblement confondu avec l'axe dudit bras (100), la première face (31) du dispositif de mesure (3) étant alors en contact avec la peau du bras (100) du porteur (102), **caractérisé en ce que** le dispositif de mesure (3) comprend :
- au moins trois capteurs de température cutanée (33) configurés pour mesurer une température cutanée du porteur (102), les capteurs de température cutanée (33) étant positionnés sur la première face (31) du dispositif de mesure (3), dans une première zone (Z33) du dispositif de mesure (3), et s'étendant sensiblement sur au moins une partie d'une ligne périphérique du dispositif de mesure (3) en configuration portée ;
- au moins un capteur de température cavitaire (34) configuré pour mesurer une température dans ladite aisselle (101) du porteur (102), le capteur de température cavitaire (34) étant agencé sur la deuxième face (32) du dispositif de mesure (3), dans une deuxième zone (Z34) du dispositif de mesure (3) ;
ladite première zone (Z33) des capteurs de température cutanée (33) et ladite deuxième zone (Z34) du ou des capteurs de température cavitaire (34) étant agencées au moins en partie en regard l'une de l'autre ou étant sensiblement adjacentes, en projection dans un plan orthogonalement auxdites première et deuxième faces (31, 32)
le dispositif de mesure (3) étant configuré pour pouvoir être dans une configuration non portée dans laquelle il est sensiblement plan
le dispositif de mesure (3) comprenant en outre au moins un capteur de température proximale (35) configuré pour mesurer une température environnante à proximité immédiate du bras (100) du porteur (102), le capteur de température proximale (35) étant agencé sur ou au voisinage de la deuxième face (32) du dispositif de mesure (3), le dispositif étant en outre **caractérisé en ce qu'**en configuration non-portée, la distance entre un capteur de température cutanée (33) et l'au moins un capteur de température proximale (35) est supérieure à 10 cm.

2. Dispositif de mesure selon la revendication 1, **caractérisé en ce que** le nombre de capteur(s) de température cavitaire (34) est inférieur au nombre de capteurs de température cutanée (33).

3. Dispositif de mesure selon la revendication 1 ou 2, **caractérisé en ce que** :
- la première face du dispositif de mesure est agencée pour être en contact direct de la peau du bras du porteur sans couche intermédiaire entre la première face et la peau du bras du porteur, et les au moins trois capteurs de température cutanée sont situés sur cette première face en étant orientés vers l'extérieur du dispositif, et
- la deuxième face du dispositif de mesure est agencée pour être en contact direct de l'aisselle du porteur sans couche intermédiaire entre la deuxième face et l'aisselle du porteur, et l'au moins un capteur de température cavitaire est situé sur cette deuxième face en étant orienté vers l'extérieur du dispositif

4. Dispositif de mesure selon l'une quelconque des revendications précédentes, **caractérisé en ce que**, dans la configuration portée, il s'étend angulairement sur au moins 90°.

5. Dispositif de mesure selon l'une quelconque des revendications précédentes, **caractérisé en ce qu'**il est souple et configuré pour pouvoir être déformé, par enroulement, entre une configuration non-portée et la configuration portée.

6. Dispositif de mesure selon l'une quelconque des revendications précédentes, **caractérisé en ce que** le capteur de température proximale (35) est décalé angulairement par rapport à l'au moins un capteur de température cavitaire (34), en configuration portée du dispositif de mesure (3), d'au moins 90°.

7. Dispositif de mesure selon l'une quelconque des revendications précédentes, **caractérisé en ce qu'**il comprend en outre au moins un capteur de données physico-chimiques complémentaires (36).

8. Dispositif de mesure selon l'une quelconque des revendications précédentes, **caractérisé en ce que** ladite première zone (Z33) dans laquelle sont agencés les capteurs de température cutanée (33) est allongée et s'étend le long d'une direction longitudinale du dispositif de mesure (3), les capteurs de température cutanée (33) étant disposés de façon alignée ou en quinconce dans ladite première zone (Z33) allongée.

9. Dispositif de mesure selon la revendication 8, **caractérisé en ce que** le au moins un capteur de température cavitaire (34) est agencé en regard de ladite première zone (Z33) des capteurs de température cutanée (33) selon la direction longitudinale.

10. Dispositif de mesure selon l'une quelconque des revendications précédentes, **caractérisé en ce qu'**il se présente sous la forme d'une bande allongée dans une direction longitudinale (X) qui, dans la configuration portée, correspond sensiblement à une ligne périphérique du dispositif de mesure (3), la bande présentant :
- une longueur (L), selon la direction (X), supérieure à 12 cm;
- une largeur (I), selon une direction qui est sensiblement parallèle à l'axe (Z) du cylindre en configuration portée, inférieure à 6 cm;
et la bande possédant un axe longitudinal médian (10), un axe transversal médian (11), un bord supérieur (12) et un bord inférieur (13).

11. Dispositif de mesure selon la revendication 10, **caractérisé en ce que** les capteurs de température cutanée (33) et/ou le au moins un capteur de température cavitaire (34) sont disposés sensiblement le long de l'axe longitudinal médian (10) de la bande.

12. Dispositif de mesure selon la revendication 10, **caractérisé en ce que** les capteurs de température cutanée (33) et/ou le au moins un capteur de température cavitaire (34) sont décalés en direction du bord supérieur (12) par rapport à l'axe longitudinal médian (10) de la bande.

13. Dispositif de mesure selon l'une des revendications 10 à 12, **caractérisé en ce qu'**il comprend :
- une nappe (15) allongée portant les capteurs de température cutanée (33) et le au moins un capteur de température cavitaire (34) ;
- un boîtier (20) contenant une carte électronique (23) et une batterie (24) connectées à la nappe (15) allongée, le boîtier portant le au moins un capteur de température proximale (35) et/ou portant le au moins un capteur de données physico-chimiques complémentaires (36) si le dispositif de de mesure (3) est conforme à la revendication 7 ;
le boîtier (20) et la nappe (15) allongée étant englobés, par exemple par surmoulage, dans une matière souple telle qu'un silicone.

14. Dispositif de mesure selon l'une quelconque des revendications précédentes, **caractérisé en ce qu'**il comprend :
- un premier jeu de capteurs (S1) comportant :
-- au moins trois capteurs de température cutanée (33-1) qui sont positionnés sur ou au voisinage de la première face (31) du dispositif de mesure (3), dans une première zone (Z33-1) du premier jeu de capteurs, et qui s'étendent sur une première longueur (L33-1);
-- au moins un capteur de température cavitaire (34-1) qui est agencé sur ou au voisinage de la deuxième face (32) du dispositif de mesure (3), dans une deuxième zone (Z34-1) du premier jeu de capteurs du dispositif de mesure (3) qui est au moins en partie en regard de la première zone (Z33-1) du premier jeu de capteurs, ou sensiblement adjacente à celle-ci, en projection dans un plan orthogonalement auxdites première et deuxième faces (31, 32) ;
- un deuxième jeu de capteurs (S2) comportant :
-- au moins trois capteurs de température cutanée (33-2) qui sont positionnés sur ou au voisinage de la deuxième face (32) du dispositif de mesure (3), dans une première zone (Z33-2) du deuxième jeu de capteurs, et qui s'étendent sur une deuxième longueur (L33-2) différente de la première longueur (L33-1);
-- au moins un capteur de température cavitaire (34-2) qui est agencé sur ou au voisinage de la première face (31) du dispositif de mesure (3), dans une deuxième zone (Z34-2) du deuxième jeu de capteurs du dispositif de mesure (3) qui est au moins en partie en regard de la première zone (Z33-2) du deuxième jeu de capteurs, ou sensiblement adjacente à celle-ci, en projection dans un plan orthogonalement auxdites première et deuxième faces (31, 32)
le dispositif étant agencé pour utiliser ou activer un seul jeu de capteurs à la fois parmi le premier jeu de capteurs et le deuxième jeu de capteurs.

15. Dispositif de mesure selon la revendication 14, **caractérisé en ce que** :
- le nombre de capteur(s) de température cavitaire (34-1) du premier jeu est inférieur au nombre de capteurs de température cutanée (33-1) du premier jeu, et
- le nombre de capteur(s) de température cavitaire (34-2) du deuxième jeu est inférieur au nombre de capteurs de température cutanée (33-2) du deuxième jeu.

16. Ensemble comportant un dispositif de mesure (3) selon l'une quelconque des revendications précédentes, **caractérisé en ce qu'**il comprend en outre un étui (27) souple, destiné à recevoir le dispositif de mesure (3), l'étui (27) possédant une fenêtre (28) en regard de chacun des capteurs du dispositif de mesure (3), l'étui (27) possédant, sur sa face (29) destinée à être en contact avec le bras (100) du porteur (102), au moins une portion adhésive (30) destinée à être collée sur le bras (100) du porteur (102).

17. Ensemble selon la revendication 16, **caractérisé en ce que** la portion adhésive (30) comprend une couche d'adhésif recouverte par au moins une bande pelable, avant la première utilisation.

18. Système de détermination d'une température interne profonde d'un être humain, **caractérisé en ce qu'**il comprend ;
- un dispositif de mesure (3) d'une pluralité de températures selon l'une des revendications 1 à 15, ou un ensemble selon l'une des revendications 16 ou 17 ;
- une unité de traitement (2) configurée et/ou programmée pour déterminer la température interne profonde du porteur (102) du dispositif de mesure (3), à partir des données de température mesurées par ledit dispositif de mesure (3).

19. Procédé de détermination d'une température interne profonde d'un être humain, mis en oeuvre au moyen d'un système selon la revendication 18, le procédé comprenant les étapes suivantes :
a) mesure d'au moins une température cutanée à un temps t ou sur une période de temps p, par l'au moins trois capteurs de température cutanée du système de détermination,
b) mesure d'au moins une température cavitaire à un temps t ou sur une période de temps p, par l'au moins un capteur de température cavitaire du dispositif de mesure du système de mesure et de détermination,
c) le dispositif de mesure envoie les données de température mesurées aux étapes a), b), par le biais d'un émetteur, à un récepteur équipant l'unité de traitement du système de mesure et de détermination selon l'invention,
d) le récepteur de l'unité de traitement reçoit les données de température mesurées et les transmet à une mémoire de l'unité de traitement qui stocke les données de température,
- l'unité de traitement compare les données de température cutanée pour chaque capteur et détermine la température cutanée du porteur pour chaque capteur pour un temps t ou une période p en utilisant par exemple la moyenne des températures mesurées pour ce capteur
- l'unité de traitement compare pour le ou chaque capteur de température cavitaire et détermine la température cavitaire pour le ou chaque capteur pour un temps t ou une période p en utilisant par exemple la moyenne de températures mesurées pour chaque capteur,
e) à partir de la donnée de température retenue pour chaque capteur de température cutanée et pour le ou chaque capteur de température cavitaire, pour un temps t ou sur au moins une période p, l'unité de traitement détermine la température interne profonde du porteur, utilisant les données de températures pour chaque capteur pour un temps t ou une période p, ou un jeu de données correspondant aux données de températures pour chaque capteur relevées pendant une période glissante d'observation P.

20. Procédé selon la revendication 19, mis en oeuvre au moyen d'un système selon la revendication 18, comprenant en outre les étapes suivantes :
- Après l'étape a), une étape a') de détermination d'une valeur unique de température cutanée, de préférence en choisissant la valeur la plus élevée parmi les températures cutanées des ou mesurées par les au moins trois capteurs cutanés (33),
- Après l'étape b), une étape b') de détermination d'une valeur unique de température cavitaire, de préférence en choisissant la valeur la plus élevée parmi les températures cavitaires de ou mesurées par l'au moins un capteur de température cavitaire (34).
- à partir de la valeur unique de température cutanée déterminée en étape a', de la valeur unique de température cavitaire déterminée en étape b', l'unité de traitement (2) détermine la température interne profonde du porteur être humain.

21. Procédé selon la revendication 20, mis en oeuvre au moyen d'un système selon la revendication 18 comprenant en outre les étapes suivantes :
- une étape FFF de mesure d'au moins une température proximale à un temps t ou sur une période de temps p, par l'au moins un capteur de température proximale (35),
- une étape FFF' de détermination d'une valeur unique de température proximale, de préférence en choisissant la valeur la plus élevée parmi les températures proximales de ou mesurées par l'au moins un capteur de température proximale (35)
- à partir de la valeur unique de température cutanée déterminée en étape a', de la valeur unique de température cavitaire déterminée en étape b', de la valeur unique de température proximale déterminée en étape FFF', l'unité de traitement (2) détermine la température interne profonde du porteur être humain.

## Patentansprüche

1. Vorrichtung zum Messen mehrerer Temperaturen zur Bestimmung einer tiefen Innentemperatur eines die Messvorrichtung tragenden Menschen (102), wobei die Messvorrichtung (3) eine erste Seite (31) und eine der ersten Seite (31) entgegengesetzte zweite Seite (32) aufweist und dazu ausgebildet ist, eine Tragekonfiguration einnehmen zu können, in welcher die Messvorrichtung (3) zumindest teilweise auf sich selbst gerollt ist und mindestens einen Teil eines Zylinders mit einer Achse (Z) bildet, wobei die erste Seite (31) der Achse (Z) zugewandt ist, wobei die Messvorrichtung (3) in ihrer Tragekonfiguration dazu vorgesehen ist, einen Arm (100) des Trägers (102) nahe einer entsprechenden Achsel (101) des Körpers des Trägers (102) zumindest teilweise zu umgeben, derart, dass die Achse (Z) der Messvorrichtung (3) im Wesentlichen mit der Achse des Arms (100) zusammenfällt, wobei die erste Seite (31) der Messvorrichtung (3) somit in Kontakt mit der Haut des Arms (100) des Trägers (102) ist, **dadurch gekennzeichnet, dass** die Messvorrichtung (3) aufweist:
- mindestens drei Hauttemperatursensoren (33), die zum Messen einer Hauttemperatur des Trägers (102) ausgebildet sind, wobei die Hauttemperatursensoren (33) auf der ersten Seite (31) der Messvorrichtung (3) in einer ersten Zone (Z33) der Messvorrichtung (3) angeordnet sind, und sich im Wesentlichen auf einem Teil einer Umfangslinie der Messvorrichtung (3) in der Tragekonfiguration erstrecken;
- mindestens einem Kavitätstemperatursensor (34), der zum Messen einer Temperatur in der Achsel (101) des Trägers (102) ausgebildet ist, wobei der Kavitätstemperatursensor (34) auf der zweiten Seite (32) der Messvorrichtung (3) in einer zweiten Zone (Z34) der Messvorrichtung (3) angeordnet ist;
wobei die erste Zone (Z33) der Hauttemperatursensoren (33) und die zweite Zone (Z34) des oder der Kavitätstemperatursensoren (34), in eine zu der ersten und der zweite Seite (31, 32) orthogonalen Ebene projiziert, zumindest teilweise einander gegenüberliegend angeordnet sind oder im Wesentlichen einander benachbart angeordnet sind,
wobei die Messvorrichtung (3) dazu ausgebildet ist, eine Nicht-Tragekonfiguration einnehmen zu können, in welcher sie im Wesentlichen eben ist,
wobei die Messvorrichtung (3) ferner mindestens einen Proximaltemperatursensor (35) aufweist, der dazu ausgebildet ist, eine Umgebungstemperatur in unmittelbarer Nähe des Arms (100) des Trägers (102) zu messen, wobei der Proximaltemperatursensor (35) auf oder nahe der zweiten Seite (32) der Messvorrichtung (3) angeordnet ist,
wobei die Vorrichtung ferner **dadurch gekennzeichnet ist, dass** in der Nicht-Tragekonfiguration der Abstand zwischen einem Hauttemperatursensor (33) und dem mindestens einen Proximaltemperatursensor (35) größer als 10 cm ist.

2. Messvorrichtung nach Anspruch 1, **dadurch gekennzeichnet, dass** die Anzahl der Kavitätstemperatursensoren (34) geringer als die Anzahl der Hauttemperatursensoren (33) ist.

3. Messvorrichtung nach Anspruch 1 oder 2, **dadurch gekennzeichnet, dass**:
- die erste Seite der Messvorrichtung zum direkten Kontakt mit der Haut des Arms des Trägers ohne Zwischenschicht zwischen der ersten Seite und der Haut des Arms des Trägers angeordnet ist, und die mindestens drei Hauttemperatursensoren auf dieser ersten Seite zur Außenseite der Vorrichtung gerichtet angeordnet sind, und
- die zweite Seite der Messvorrichtung zum direkten Kontakt mit der Achsel des Trägers ohne Zwischenschicht zwischen der ersten Seite und der Achsel des Trägers angeordnet ist, und der mindestens eine Kavitätstemperatursensor auf dieser zweiten Seite zur Außenseite der Vorrichtung gerichtet angeordnet ist.

4. Messvorrichtung nach einem der vorangehenden Ansprüche, **dadurch gekennzeichnet, dass** sie sich in der Tragekonfiguration winkelmäßig über mindestens 90° erstreckt.

5. Messvorrichtung nach einem der vorangehenden Ansprüche, **dadurch gekennzeichnet, dass** sie biegsam ist und durch Einrollen zwischen einer Nicht-Tragekonfiguration und der Tragekonfiguration verformbar ist.

6. Messvorrichtung nach einem der vorangehenden Ansprüche, **dadurch gekennzeichnet, dass** der Proximaltemperatursensor (35) in Bezug auf den mindestens einen Kavitätstemperatursensor (34) in der Tragekonfiguration der Messvorrichtung (33) winkelmäßig um mindestens 90° versetzt ist.

7. Messvorrichtung nach einem der vorangehenden Ansprüche, **dadurch gekennzeichnet, dass** sie ferner mindestens einen Sensor (36) für komplementäre physikochemische Daten aufweist.

8. Messvorrichtung nach einem der vorangehenden Ansprüche, **dadurch gekennzeichnet, dass** die erste Zone (Z33), in welcher die Hauttemperatursensoren (33) angeordnet ist, langgestreckt ist und sich entlang einer Längsrichtung der Messvorrichtung (3) erstreckt, wobei die Hauttemperatursensoren (33) ausgerichtet oder gestaffelt in der langgestreckten ersten Zone (Z33) angeordnet sind.

9. Messvorrichtung nach Anspruch 8, **dadurch gekennzeichnet, dass** der mindestens eine Kavitätstemperatursensor (34) in Längsrichtung gegenüber der ersten Zone (Z33) der Hauttemperatursensoren (33) angeordnet ist.

10. Messvorrichtung nach einem der vorangehenden Ansprüche, **dadurch gekennzeichnet, dass** sie in Form eines in Längsrichtung (X) langgestreckten Bandes vorliegt, die in der Tragekonfiguration im Wesentlichen einer Umfangsrichtung der Messvorrichtung (3) entspricht, wobei das Band aufweist:
- eine Länge (L) von mehr als 12 cm in der Richtung (X);
- eine Breite (I) von weniger als 6 cm in einer Richtung, die im Wesentlichen parallel zur Achse (Z) des Zylinders in der Tragekonfiguration ist;
und wobei das Band eine Längsmittelachse (10), eine Quermittelachse (11), einen oberen Rand (12) und einen unteren Rand (13) aufweist.

11. Messvorrichtung nach Anspruch 10, **dadurch gekennzeichnet, dass** die Hauttemperatursensoren (33) und/oder der mindestens eine Kavitätstemperatursensor (34) im Wesentlichen entlang der Längsmittelachse (10) des Bandes angeordnet sind.

12. Messvorrichtung nach Anspruch 10, **dadurch gekennzeichnet, dass** die Hauttemperatursensoren (33) und/oder der mindestens eine Kavitätstemperatursensor (34) in Richtung des oberen Randes (12) in Bezug auf die Längsmittelachse (10) des Bandes versetzt sind.

13. Messvorrichtung nach einem der Ansprüche 10 bis 12, **dadurch gekennzeichnet, dass** sie aufweist:
- eine langgestreckte Bahn (15), welche die Hauttemperatursensoren (33) und/oder den mindestens einen Kavitätstemperatursensor (34) trägt;
- ein Gehäuse (20), das eine elektronische Karte (23) und eine Batterie (24) enthält, welche mit der langgestreckten Bahn (15) verbunden sind, wobei das Gehäuse den mindestens einen Proximaltemperatursensor (35) und/oder den mindestens einen Sensor (36) für komplementäre physikochemische Daten trägt, wenn die Messvorrichtung (3) mit dem Anspruch 7 konform ist:
wobei das Gehäuse (20) und die langgestreckte Bahn (15) in einem flexiblem Material wie Silikon zum Beispiel durch Umspritzen eingeschlossen sind.

14. Messvorrichtung nach einem der vorangehenden Ansprüche, **dadurch gekennzeichnet, dass** sie aufweist:
- einen ersten Sensorsatz (S1) mit:
- mindestens drei Hauttemperatursensoren (33-1), die auf oder nahe der ersten Seite (31) der Messvorrichtung (3) in einer ersten Zone (Z33-1) des ersten Sensorsatzes angeordnet sind, und die sich über eine erste Länge (L33-1) erstrecken;
- mindestens einem Kavitätstemperatursensor (34-1), der auf oder nahe der zweiten Seite (32) der Messvorrichtung (3) in einer zweiten Zone (Z34-1) des ersten Sensorsatzes der Messvorrichtung (3) angeordnet sind, die in eine zu der ersten und der zweite Seite (31, 32) orthogonalen Ebene projiziert, zumindest teilweise der ersten Zone (Z33-1) des ersten Sensorsatzes gegenüberliegend angeordnet oder im Wesentlichen einander benachbart angeordnet ist;
- einem zweiten Sensorsatz (S2) mit:
- mindestens drei Hauttemperatursensoren (33-2), die auf oder nahe der zweiten Seite (32) der Messvorrichtung (3) in einer ersten Zone (Z33-2) des zweiten Sensorsatzes angeordnet sind, und die sich über eine zweite Länge (L33-2) erstrecken, die von der ersten Länge (L33-1) verschieden ist;
- mindestens einem Kavitätstemperatursensor (34-2), der auf oder nahe der ersten Seite (31) der Messvorrichtung (3) in einer zweiten Zone (Z34-2) des zweiten Sensorsatzes der Messvorrichtung (3) angeordnet sind, die in eine zu der ersten und der zweite Seite (31, 32) orthogonalen Ebene projiziert, zumindest teilweise der ersten Zone (Z33-2) des zweiten Sensorsatzes gegenüberliegend angeordnet oder im Wesentlichen einander benachbart angeordnet ist;
wobei die Vorrichtung dazu ausgebildet ist, unter dem ersten Sensorsatz und dem zweiten Sensorsatz nur einen einzigen Sensorsatz gleichzeitig zu verwenden oder zu aktivieren.

15. Messvorrichtung nach Anspruch 14, **dadurch gekennzeichnet, dass**:
- die Anzahl der Kavitätstemperatursensoren (34-1) des ersten Sensorsatzes geringer als die Anzahl der Hauttemperatursensoren (33-1) des ersten Satzes ist, und
- die Anzahl der Kavitätssensoren (34-2) des zweiten Satzes geringer als die Anzahl der Hauttemperatursensoren (33-2) des zweiten Satzes ist.

16. Anordnung mit einer Messvorrichtung (3) nach einem der vorhergehenden Ansprüche, **dadurch gekennzeichnet, dass** sie ferner eine biegsame Hülle (27) aufweist, die zum Aufnehmen der Messvorrichtung (3) vorgesehen ist, wobei die Hülle (27) ein Fenster (28) gegenüber jedem der Sensoren der Messvorrichtung (3) aufweist, wobei die Hülle (27) auf ihrer für den Kontakt mit dem Arm (100) des Trägers (102) vorgesehenen Seite mindestens eine klebenden Bereich (30) aufweist, welcher zum Kleben auf den Arm (100) des Trägers (102) vorgesehen ist.

17. Anordnung nach Anspruch 16, **dadurch gekennzeichnet, dass** der klebende Bereich (30) eine Kleberschicht aufweist, welche vor dem ersten Gebrauch durch mindestens ein abziehbares Band bedeckt ist.

18. System zur Bestimmung einer tiefen Innentemperatur eines Menschen, **dadurch gekennzeichnet, dass** es aufweist:
- eine Vorrichtung (3) zum Messen mehrere Temperaturen nach einem der Ansprüche 1 bis 15 oder eine Anordnung nach einem der Ansprüche 16 oder 17;
- Verarbeitungseinheit (2) die dazu ausgebildet und/oder programmiert ist, die tiefe Innentemperatur des Trägers (102) der Messvorrichtung (3) anhand der von der Messvorrichtung (3) gemessenen Temperaturdaten zu bestimmen.

19. Verfahren zum Bestimmen einer tiefen Innentemperatur eines Menschen, implementiert mittels eines Systems nach Anspruch 18, wobei das Verfahren die folgenden Schritte aufweist:
a) Messen mindestens einer Hauttemperatur zu einem Zeitpunkt t oder über einen Zeitraum p durch mindestens drei Hauttemperatursensoren des Bestimmungssystems,
b) Messen mindestens einer Kavitätstemperatur zu einem Zeitpunkt t oder über einen Zeitraum p durch den mindestens einen Kavitätstemperatursensor der Messvorrichtung des Mess- und Bestimmungssystems,
c) Senden der in den Schritten a), b) gemessenen Temperaturdaten durch die Messvorrichtung mittels eines Senders an einen Empfänger, der mit der Verarbeitungseinheit des Mess- und Bestimmungssystems gemäß der Erfindung versehen ist,
d) Empfangen der gemessenen Temperaturdaten durch den Empfänger und Übertragen derselben an einen Speicher der Verarbeitungseinheit, welche die Temperaturdaten speichert,
- durch die Verarbeitungseinheit erfolgendes Vergleichen der Hauttemperaturdaten jedes Sensors und Bestimmen der Hauttemperatur des Trägers für jeden Sensor für einen Zeitpunkt t oder einen Zeitraum p beispielsweise unter Verwendung des Mittelwerts der gemessenen Temperaturen dieses Sensors,
- durch die Verarbeitungseinheit erfolgendes Vergleichen der Kavitätstemperaturdaten des oder jedes Sensors und Bestimmen der Kavitätstemperatur des Trägers für jeden Sensor für einen Zeitpunkt t oder einen Zeitraum p beispielsweise unter Verwendung des Mittelwerts der gemessenen Temperaturen jedes Sensors,
e) basierend auf den für jeden Hauttemperatursensor oder den oder jeden der Kavitätstemperatursensoren gespeicherten Temperaturdaten für einen Zeitpunkt t oder einen Zeitraum p, Bestimmen der tiefen Innentemperatur des Trägers durch die Verarbeitungseinheit unter Verwendung der Temperaturdaten jedes Sensors für einen Zeitpunkt t oder einen Zeitraum p oder eines Datensatzes, der den Temperaturdaten entspricht, welche über einen gleitenden Beobachtungszeitraum P erhoben wurden.

20. Verfahren nach Anspruch 19, implementiert durch ein System nach Anspruch 18, ferner mit den folgenden Schritten:
- nach dem Schritt a), einem Schritt a') des Bestimmens eines Einzelwerts der Hauttemperatur, vorzugsweise durch Wählen des höchsten Werts unter den Hauttemperaturen der mindestens drei Hautsensoren (33) oder den von diesen gemessenen Hauttemperaturen,
- nach dem Schritt b), einem Schritt b') des Bestimmens eines Einzelwerts der Kavitätstemperatur, vorzugsweise durch Wählen des höchsten Werts unter den Kavitätstemperaturen des Kavitätstemperatursensors (34) oder den von diesem gemessenen Kavitätstemperaturen,
- basierend auf dem im Schritt a' bestimmten Einzelwert der Hauttemperatur, dem im Schritt b' bestimmten Einzelwert der Kavitätstemperatur, Bestimmen der tiefen Innentemperatur des menschlichen Trägers durch die Verarbeitungseinheit (2).

21. Verfahren nach Anspruch 20, implementiert durch ein System nach Anspruch 18, ferner mit den folgenden Schritten:
- einem Schritt FFF des Messens mindestens einer Proximaltemperatur zu einem Zeitpunkt t oder über einen Zeitraum p durch den mindestens einen Proximaltemperatursensor (35),
- einem Schritt FFF' des Bestimmens eines Einzelwerts der Proximaltemperatur, vorzugsweise durch Wählen des höchsten Werts unter den Proximaltemperaturen des Proximaltemperatursensors (35) oder den von diesem gemessenen Proximaltemperaturen,
- basierend auf dem im Schritt a' bestimmten Einzelwert der Hauttemperatur, dem im Schritt b' bestimmten Einzelwert der Kavitätstemperatur, dem im Schritt FFF' bestimmten Einzelwert der Proximaltemperatur, Bestimmen der tiefen Innentemperatur des menschlichen Trägers durch die Verarbeitungseinheit (2).

## Claims

1. Device for measuring a plurality of temperatures with the aim of determining a core internal temperature of a human being (102) wearing said measurement device, the measurement device (3) having a first face (31) and a second face (32) opposite the first face (31) and being configured to be able to be in a worn configuration in which the measurement device (3) is at least partially wound over itself and forms at least one cylindrical portion having an axis (Z), the first face (31) being turned towards the axis (Z), the measurement device (3), in its worn configuration, being intended to at least partially encircle an arm (100) of the wearer (102), in the vicinity of a corresponding armpit (101) of the body of the wearer (102), so that the axis (Z) of the measurement device (3) is substantially merged with the axis of said arm (100), the first face (31) of the measurement device (3) then being in contact with the skin of the arm (100) of the wearer (102), **characterized in that** the measurement device (3) comprises:
- at least three skin temperature sensors (33) configured to measure a skin temperature of the wearer (102), the skin temperature sensors (33) being positioned on the first face (31) of the measurement device (3), in a first zone (Z33) of the measurement device (3), and extending substantially over at least part of a peripheral line of the measurement device (3) in the worn configuration;
- at least one cavity temperature sensor (34) configured to measure a temperature in said armpit (101) of the wearer (102), the cavity temperature sensor (34) being arranged on the second face (32) of the measurement device (3), in a second zone (Z34) of the measurement device (3);
said first zone (Z33) of the skin temperature sensors (33) and said second zone (Z34) of the cavity temperature sensor or sensors (34) being arranged at least partly facing each other or being substantially adjacent, projecting in a plane orthogonal to said first and second faces (31, 32)
the device (3) being configured to be able to be in an unworn configuration in which it is substantially flat
the device also comprising at least one proximal temperature sensor (35) configured to measure a surrounding temperature in the immediate vicinity of the arm (100) of the wearer (102), the proximal temperature sensor (35) being arranged on or near the second face (32) of the device (3),
the device also being **characterized in that**, in the unworn configuration, the distance between a skin temperature sensor (33) and the at least one proximal temperature sensor (35) is greater than 10 cm.

2. Measurement device according to claim 1, **characterized in that** the number of cavity temperature sensor(s) (34) is less than the number of skin temperature sensors (33).

3. Measurement device according to claim 1 or 2, **characterized in that**:
- the first face of the measurement device is arranged to be in direct contact with the skin of the arm of the wearer without an intermediate layer between the first face and the skin of the arm of the wearer, and the at least three skin temperature sensors are situated on this first face, wherein they are oriented towards the exterior of the device, and
- the second face of the measurement device is arranged to be in direct contact with the armpit of the wearer without an intermediate layer between the second face and the armpit of the wearer, and the at least one cavity temperature sensor is situated on this second face, wherein it is oriented towards the exterior of the device.

4. Measurement device according to any one of the preceding claims, **characterized in that**, in the worn configuration, it extends angularly over at least 90°.

5. Measurement device according to any one of the preceding claims, **characterized in that** it is flexible and configured to be able to be deformed, by winding, between an unworn configuration and the worn configuration.

6. Measurement device according to any one of the preceding claims, **characterized in that** the proximal temperature sensor (35) is angularly offset with respect to the at least one cavity temperature sensor (34), in the worn configuration of the measurement device (3), by at least 90°.

7. Measurement device according to any one of the preceding claims, **characterized in that** it also comprises at least one additional physicochemical data sensor (36).

8. Measurement device according to any one of the preceding claims, **characterized in that** said first zone (Z33), in which the skin temperature sensors (33) are arranged, is elongated and extends in a longitudinal direction of the measurement device (3), the skin temperature sensors (33) being arranged in an aligned manner or staggered in said first elongated zone (Z33).

9. Measurement device according to claim 8, **characterized in that** the at least one cavity temperature sensor (34) is arranged facing said first zone (Z33) of the skin temperature sensors (33) in the longitudinal direction.

10. Measurement device according to any one of the preceding claims, **characterized in that** it is in the form of a band elongated in a longitudinal direction (X) which, in the worn configuration, substantially corresponds to a peripheral line of the measurement device (3), the band having:
- a length (L), in the direction (X), greater than 12 cm;
- a width (I), in a direction which is substantially parallel to the axis (Z) of the cylinder in the worn configuration, of less than 6 cm;
and the band having a longitudinal central axis (10), a transverse central axis (11), an upper edge (12) and a lower edge (13).

11. Measurement device according to claim 10, **characterized in that** the skin temperature sensors (33) and/or the at least one cavity temperature sensor (34) are arranged substantially along the longitudinal central axis (10) of the band.

12. Measurement device according to claim 10, **characterized in that** the skin temperature sensors (33) and/or the at least one cavity temperature sensor (34) are offset in the direction of the upper edge (12) with respect to the longitudinal central axis (10) of the band.

13. Measurement device according to one of claims 10 to 12, **characterized in that** it comprises:
- an elongated tape (15) bearing the skin temperature sensors (33) and the at least one cavity temperature sensor (34);
- a case (20) containing an electronic board (23) and a battery (24) connected to the elongated tape (15), the case bearing the at least one proximal temperature sensor (35) and/or bearing the at least one additional physicochemical data sensor (36), if the measurement device (3) is according to claim 7;
the case (20) and the elongated tape (15) are covered, for example by over-moulding, in a flexible material such as a silicone.

14. Measurement device according to any one of the preceding claims, **characterized in that** it comprises:
- a first set of sensors (51) containing:
-- at least three skin temperature sensors (33-1), which are positioned on or near the second face (31) of the measurement device (3), in a first zone (Z33-1) of the first set of sensors, and which extend over a first length (L33-1);
-- at least one cavity temperature sensor (34-1), which is arranged on or near the second face (32) of the measurement device (3), in a second zone (Z34-1) of the first set of sensors of the measurement device (3) which is at least partly facing the first zone (Z33-1) of the first set of sensors, or substantially adjacent to it, projecting in a plane orthogonal to said first and second faces (31, 32);
- a second set of sensors (S2) containing:
-- at least three skin temperature sensors (33-2), which are positioned on or near the second face (32) of the measurement device (3), in a first zone (Z33-2) of the second set of sensors, and which extend over a second length (L33-2) different from the first length (L33-1);
-- at least one cavity temperature sensor (34-2), which is arranged on or near the first face (31) of the measurement device (3), in a second zone (Z34-2) of the second set of sensors of the measurement device (3) which is at least partly facing the first zone (Z33-2) of the second set of sensors, or substantially adjacent to it, projecting in a plane orthogonal to said first and second faces (31, 32) the device being arranged to use or activate a single set of sensors at once from the first set of sensors and the second set of sensors.

15. Measurement device according to claim 14, **characterized in that**:
- the number of cavity temperature sensor(s) (34-1) of the first set is less than the number of skin temperature sensors (33-1) of the first set, and
- the number of cavity temperature sensor(s) (34-2) of the second set is less than the number of skin temperature sensors (33-2) of the second set.

16. Assembly containing a measurement device (3) according to any one of the preceding claims, **characterized in that** it comprises, moreover, a flexible sheath (27), intended to receive the measurement device (3), the sheath (27) having a window (28) facing each of the sensors of the measurement device (3), the sheath (27) having, on its face (29) intended to be in contact with the arm (100) of the wearer (102), at least one adhesive portion (30) intended to be stuck on the arm (100) of the wearer (102).

17. Assembly according to claim 16, **characterized in that** the adhesive portion (30) comprises an adhesive layer covered by at least one band that can be peeled off before the first use.

18. System for determining a core internal temperature of a human being, **characterized in that** it comprises;
- a device (3) for measuring a plurality of temperatures according to one of claims 1 to 15, or an assembly according to one of claims 16 or 17;
- a processing unit (2) configured and/or programmed to determine the core internal temperature of the wearer (102) of the measurement device (3), based on the temperature data measured by said measurement device (3).

19. Method for determining a core internal temperature of a human being, implemented by means of a system according to claim 18, the method comprising the following steps:
a) measuring at least one skin temperature at a time t or over a period of time p, by the at least three skin temperature sensors of the determination system,
b) measuring at least one cavity temperature at a time t or over a period of time p, by the at least one cavity temperature sensor of the measurement device of the measurement and determination system,
c) the measurement device sends the temperature data measured in steps a), b), through a transmitter, to a receiver equipping the processing unit of the measurement and determination system according to the invention,
d) the receiver of the processing unit receives the measured temperature data and transmits them to a memory of the processing unit, which stores the temperature data,
- the processing unit compares the skin temperature data for each sensor and determines the skin temperature of the wearer for each sensor for a time t or a period p using for example the average of the temperatures measured for this sensor,
- the processing unit compares, for the or each cavity temperature sensor, and determines, for the or each sensor, the cavity temperature for a time t or a period p using for example the average of the temperatures measured for each sensor,
e) based on the item of temperature data retained for each skin temperature sensor and for the or each cavity temperature sensor, for a time t or over at least one period p, the processing unit determines the core internal temperature of the wearer, using the temperature data for each sensor for a time t or a period p, or a set of data corresponding to the temperature data for each sensor collected during a rolling observation period P.

20. Method according to claim 19, implemented by means of a system according to claim 18, also comprising the following steps:
- after step a), a step a') of determining a single skin temperature value, preferably by selecting the highest value from among the skin temperatures from or measured by the at least three skin sensors (33),
- after step b), a step b') of determining a single cavity temperature value, preferably by selecting the highest value from among the cavity temperatures from or measured by the at least one cavity temperature sensor (34),
- based on the single skin temperature value determined in step a' and the single cavity temperature value determined in step b', the processing unit (2) determines the core internal temperature of the human wearer.

21. Method according to claim 20, implemented by means of a system according to claim 18, also comprising the following steps:
- a step FFF of measuring at least one proximal temperature at a time t or over a period of time p, by the at least one proximal temperature sensor (35),
- a step FFF' of determining a single proximal temperature value, preferably by selecting the highest value from among the proximal temperatures from or measured by the at least one proximal temperature sensor (35),
- based on the single skin temperature value determined in step a', the single cavity temperature value determined in step b' and the single proximal temperature value determined in step FFF', the processing unit (2) determines the core internal temperature of the human wearer.
